(19) Europäisches Patentamt
European Patent Office
Office européen des brevets

(11) **EP 0 582 148 B1**

(12) **EUROPÄISCHE PATENTSCHRIFT**

(45) Veröffentlichungstag und Bekanntmachung des Hinweises auf die Patenterteilung:
**17.03.1999 Patentblatt 1999/11**

(51) Int Cl.⁶: **G02B 23/24**, G02B 21/22, G02B 27/22

(21) Anmeldenummer: **93111804.6**

(22) Anmeldetag: **23.07.1993**

(54) **Endoskopischer Vorsatz für ein stereoskopisches Beobachtungssystem**

Endoscopic attachment for stereoscopic viewing system

Adaptateur endoscopique pour système d'observation stéréoscopique

(84) Benannte Vertragsstaaten:
**CH DE LI**

(30) Priorität: **01.08.1992 DE 4225507**
**21.01.1993 DE 4301466**

(43) Veröffentlichungstag der Anmeldung:
**09.02.1994 Patentblatt 1994/06**

(73) Patentinhaber: **Carl Zeiss**
**D-89520 Heidenheim (Brenz) (DE)**

(72) Erfinder:
• **Strähle, Fritz, Dr.**
**D-73540 Heubach-Lautern (DE)**
• **Vry, Uwe, Dr.**
**D-73432 Aalen (DE)**
• **Sander, Ulrich, Dr.**
**D-73447 Oberkochen (DE)**

(56) Entgegenhaltungen:
**EP-A- 0 019 792**     **US-A- 3 486 806**
**US-A- 5 122 650**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein stereoskopisches Endoskop.

Es ist bereits bekannt, für chirurgische Eingriffe in engen und tiefen Körperhöhlen flexible oder starre Endoskope einzusetzen. In den meisten Fällen werden die Betrachtungskanäle durch Bildleiter repräsentiert. Die Übertragung der Bilder über Bildleiter weist jedoch gravierende Nachteile bezüglich der Farbqualität und der erreichten Auflösung auf. Diese Nachteile sind bei starren Endoskopen vermieden.

Ein exaktes chirurgisches Arbeiten erfordert häufig einen räumlichen Bildeindruck. Es sind starre Endoskopausführungen in einer Vielzahl von Systemen unterschiedlicher Baulängen und Durchmesser bekannt, wie auch stereoskopische Systeme, z.B. nach DE-A-17 66 803, DE-U-19 96 605 sowie US-A-4 061 135. Diese bekannten Systeme weisen einen langgestreckten Grundkörper auf, in dem zur stereoskopischen Beobachtung oder Fotografie zwei parallele Optiken angeordnet sind. Die Verwendung getrennter Optiken für die stereo-Teilstrahlengänge führt jedoch bei einigen Ausführungen zusammen mit dem Instrumenten- und Beleuchtungskanal auf einen Gesamtrohrdurchmesser von 25-30mm, was den Anwendungsbereich solcher Endoskope stark einschränkt.

Für eine gute stereoskopische Abbildung müssen die beiden Beobachtungskanäle aufwendig zueinander justiert werden, nämlich:

a) Bezüglich der Bildlage, wobei die Lage der beiden Bilder durch die gegebenen Brechzahl-, Dicken- und Radientoleranzen unterschiedlich ist, aber nur innerhalb enger Grenzen variieren darf.

b) Bezüglich des Binokularfehlers, wobei die optischen Achsen der beiden Beobachtungskanäle mit einer Abweichung von wenigen Winkelminuten streng parallel zueinander ausgerichtet sein müssen.

Die erforderliche Justierung bei solchen Systemen müßte idealerweise innerhalb der Endoskoprohre vorgenommen werden, was aber kaum zu praktizieren ist, da die Endoskoprohre für Manipulationen nicht ohne weiteres zugänglich sind.

Für starre Endoskopröhren wird im Dokument EP 0 019 792 B1 vorgeschlagen, in ein Operationsmikroskop anstelle des langbrennweitigen Hauptobjektives ein stabförmiges Zusatzobjektiv einzusetzen. Mit einem Prismensystem wird bei dieser vorbekannten Lösung die große Stereobasis des Operationsmikroskops der um ungefähr den Faktor 10 kleineren Stereobasis des Zusatzobjektivs angepaßt. Nachteilig bei dieser vorbekannten Lösung ist, daß der Stereowinkel und damit auch der Stereoeindruck um denselben Faktor 10 reduziert wird. Für praktische Anwendungen, z.B. in der Laparoskopie, sind möglichst große Objektfelddurchmesser erforderlich, was zwangsläufig den Stereowinkel noch weiter reduziert, wie später noch genauer beschrieben wird. Deshalb kann die eigentliche Aufgabe einer stereoskopischen Abbildung mit diesem vorbekannten Vorschlag nicht befriedigend gelöst werden.

Ein weiterer Nachteil dieser vorbekannten Lösung besteht darin, daß die großen Durchmesser der beiden Stereostrahlengänge des Operationsmikroskopes (Gerätepupillen) von ungefähr 16 mm allein mit dem Prismensystem an die wiederum um ungefähr den Faktor 10 im Durchmesser kleineren Stereostrahlengänge des Zusatzobjektivs angepaßt werden und damit unvertretbar starke Vignettierungen mit allen allgemein bekannten Nachteilen für die Abbildungseigenschaften auftreten. Eine Verstärkung dieses Vignettierungseffekts tritt auch dadurch auf, daß die Gerätepupillen des Operationsmikroskopes nicht mit den Austrittspupillen des Zusatzobjektivs identisch sind.

In der US-PS 5 122 650 wird als Lösung für ein optisches System zur Darstellung dreidimensionaler endoskopischer Bilder vorgeschlagen, das Objekt mit einem Hauptobjektiv nach unendlich abzubilden und hinter dem Hauptobjekiv für die beiden stereoskopischen Abbildungsstrahlengänge je eine Abbildungsoptik zur Erzeugung von zwei nebeneinanderliegenden Stereohalbbildern als Zwischenbilder im Endlichen vorzusehen. Diese Zwischenbilder werden entweder auf zwei CCD-Chips abgebildet und über eine Video-Elektronik auf einen Monitor übertragen und mit einer Spezialbrille betrachtet, oder sie werden über eine Stablinsenoptik zur Beobachtung in eine Betrachtungsebene übertragen. Nachteilig bei dieser Lösung ist, daß durch die Abbildung von zwei in einer Ebene nebeneinanderliegenden stereohalbbildern ein großer Durchmesser für das optische System erforderlich ist. Soll von einem Objekt ein Zwischenbild der Größe b übertragen werden, ist dazu ein Durchmesser des optischen Systems von mindestens 2b erforderlich.

In der DE-41 16 810 A1 wird die Verwendung eines Operationsmikroskopes mit einem Endoskop vorgeschlagen. Es soll dabei dem Operationsmikroskop ein Endoskop derart vorgeschaltet werden, daß zwischen dem Hauptobjektiv des Operationsmikroskopes und dem Okular eines Endoskopes ein scherenartiges Gelenkstück angeordnet ist. Die Gelenke sollen selbsthemmend ausgeführt sein, so daß sie in jeder Bildlage, in die sie durch den Arzt gebracht werden, stehen bleiben. Es werden bei dieser Vorrichtung weder Angaben darüber gemacht, ob die Endoskopoptik überhaupt ein dreidimensionales Bild mit einer befriedigenden Stereobasis liefert, noch darüber, wie eine stereoskopische Bildübertragung vom Endoskop auf das Operationsmikroskop stattfinden soll. Es muß deshalb bezweifelt werden, ob der Chirurg ein brauchbares dreidimensionales Bild durch diese Vorrichtung erhält.

Aufgabe der Erfindung ist es, den Rohrdurchmesser eines stereoskopischen Endoskopes möglichst klein zu halten und dennoch einen guten Stereoeindruck mit guter Abbildungsqualität sowie Auflösung und Tiefenschärfe zu gewährleisten, wobei die optischen Anforderungen bezüglich Bildgröße, Pupillendurchmesser und Stereobasis durch den verwendeten Rohrdurchmesser vorgegeben sind.

Die Aufgabe wird durch ein Endoskop mit den Merkmalen des Anspruchs 1 gelöst. Bevorzugte Weiterbildungen sind in den abhängigen Ansprüchen beschrieben.

Das erfindungsgemäße System umfaßt demnach einen stabförmigen Vorsatz, der mit einem stereoskopischen Beobachtungssystem zusammenwirkt. Der endoskopische Vorsatz enthält ein für beide stereoskopischen Strahlengänge gemeinsames optisches Abbildungssystem mit mit einem freien Durchmesser D. Diese Abbildungsoptik erzeugt ein Zwischenbild des zu beobachtenden Objektes. Außerdem ist ein weiteres Abbildungssystem vorgesehen, durch das die Gerätepupillen und die Stereobasis des Beobachtungssystems derart verkleinert in den Vorsatz abgebildet sind, daß die Summe aus dem Bild der Gerätepupillen des Beobachtungssystems und dem Bild der Stereobasis des Beobachtungssystemes dem freien Durchmesser D der Abbildungsoptik innerhalb des Vorsatzes entspricht. Gleichzeitig bildet das weitere Abbildungssystem das Zwischenbild nach unendlich ab. Wie bei einem Stereomikroskop entstehen zwei Stereohalbbilder des Zwischenbildes im Beobachtungssystem.

Ist das Objekt mit einem Abbildungsmaßstab β in das Zwischenbild abgebildet, so ist beim erfindungsgemäßen Stereo-Endoskop der objektseitige Stereowinkel proportional zum beobachtungsseitigen Stereowinkel und umgekehrt proportional zu diesem Abbildungsmaßstab. Der Stereowinkel, unter dem das Zwischenbild erzeugt ist, bleibt bei der Einkopplung ins Beobachtungssystem erhalten.

Vorteilhaft ist es, wenn das Objekt innerhalb des Vorsatzes und vor der Zwischenbildebene ein weiteres mal zwischenabgebildet ist. Der Endoskop-Vorsatz erlaubt dann in Verbindung mit einem Operationsmikroskop als stereoskopisches Beobachtungssystem die aufrechte und seitenrichtige stereoskopische Objektbeobachtung. Dazu kann das Abbildungssystem aus einem Endoskop-Objektiv und einem Übertragungssystem bestehen, wobei das Übertragungssystem das vom Endoskop-Objektiv erzeugte Zwischenbild in die Zwischenbildebene und gleichzeitig die Eintrittspupille des Endoskop-Objektivs in das Bild der Gerätepupille abbildet. Für längere Endoskop-Vorsätze können darüber hinaus weitere Zwischenabbildungen vorgesehen sein.

Das weitere Abbildungssystem stellt zusammen mit einem Teil der Abbildungsoptik im Endoskop-Vorsatz ein umgekehrtes Quasi-Keplersches Fernrohr dar. Durch dieses umgekehrte Fernrohr wird der Durchmesser der Gerätepupille im selben Verhältnis wie die Stereobasis verkleinert. Damit werden die notwendigen Bedingungen sowohl bezüglich der Größen des Bildes als auch der der Pupillen erfüllt. Somit kann der gesamte verflochtene Strahlengang ohne Vignettierung von Bild und Pupille durch das enge Rohr des Vorsatzes übertragen werden. Dabei können auch mehrere Strahlengänge, z.B. zwei Strahlengänge für einen Hauptbeobachter, zwei Strahlengänge für einen Mitbeobachter und weitere Dokumentationsstrahlengänge in einem einzigen Rohr durch eine einzige Optik übertragen werden. Die Verflechtung der Strahlengänge wirkt sich nicht störend aus. Eine derartige Übertragung mehrerer Beobachtungskanäle war nach dem Stand der Technik bisher nicht möglich.

Die mit der Erfindung erzielten Vorteile bestehen insbesondere darin, daß zur Abbildung des Objektes und zur Übertragung des Zwischenbildes ein beiden stereoskopischen Strahlengängen gemeinsames optisches System benutzt wird, daß der Durchmesser der optischen Elemente im Vergleich zum Stand der Technik kleiner ist und daß die endoskopspezifischen Parameter wie z.B. Rohrdurchmesser, Rohrlänge, Stereowinkel, Tiefenschärfe, Objektfelddurchmesser, Auflösungsvermögen und Arbeitsabstand den jeweiligen Erfordernissen für verschiedene Anwendungen rechnerisch angepaßt werden können. Ein weiterer Vorteil liegt darin, daß eine Justierung von Bildlage und Binokularfehler von zwei Stereohalbbildern, wie sie bei den vorbekannten Systemen mit getrennten Optiken für die Stereokanäle erforderlich ist, entfällt. Von großem Vorteil ist außerdem, daß die Stereobasis der Gerätepupillen proportional der Brennweite des betrachtungsseitigen Objektives des umgekehrten Fernrohres verkleinert werden kann, ohne daß dadurch der Stereoeindruck verschlechtert wird. Beeindruckend ist auch die mit der Erfindung erreichbare Reduzierung des Durchmessers der optischen Elemente und damit des Endoskoprohres. Während bei dem in der US-PS 5 122 650 beschriebenen optischen System für die Übertragung eines 3 mm großen Zwischenbildes ein Durchmesser der optischen Elemente von 6 mm erforderlich ist, benötigt man für die Übertragung eines gleich großen Zwischenbildes mit dem erfindungsgemäßen optischen System nur einen Durchmesser von 3 mm. Das bedeutet, bei gleich großem Durchmesser der optischen Elemente ist die mit dem optischen System gemäß vorliegender Erfindung übertragene Bildinformation viermal größer als bei der Bildübertragung mit dem aus der US-PS 5 122 650 vorbekannten optischen System.

In einem vorteilhaften Ausführungsbeispiel wird das innerhalb des Vorsatzes erzeugte Zwischenbild durch ein Hauptobjektiv, das Bestandteil des weiteren Abbildungssystems ist, in die stereoskopischen Teilbilder nach unendlich abgebildet. Diese stereoskopische Abbildung geschieht bei visueller Beobachtung über die beiden Beobachtungskanäle des Tubus mit Okularen. Zwischen Tubus und Objektiv kann sich noch ein Vergrößerungssystem (Galilei-Wechsler oder Fernrohr-Pankrat) oder ein optischer Teiler zum Anschluß weiterer Beobachtungs- und Dokumentationsmittel (Kleinbild, TV, 3DTV) befinden.

Diese stereoskopische Abbildung kann aber auch über zwei räumlich getrennte aber identisch aufgebaute Abbildungskanäle eines stereoskopischen TV-Aufzeichnungssystems erfolgen.

Das Verhältnis des Abstands 2(A'+p') der beiden stereoskopischen Abbildungskanäle (Stereobasis) zur Brennweite des Hauptobjektivs bestimmt den für den Stereoeindruck maßgebenden Stereowinkel. Man erhält also bei kleinerer Brennweite mit entsprechend kleinerer Stereobasis denselben Stereowinkel und damit auch Stereoeindruck. Beim Stereo-TV-Endoskop erreicht man damit Abmessungen des Objektivs, die eine Integration des Objektivs in das stabförmige optische System mit den daraus resultierenden Vorteilen der Kompaktheit und der Rohrverlängerung gestattet.

Die erfindungsgemäße Lösung ermöglicht auch eine optimale optische Korrektion des gesamten Abbildungssystems. Bezüglich der optischen Abbildungseigenschaften (Auflösung, Farbtreue, Tiefenschärfe, Stereoeindruck) ist das erfindungsgemäße System mit den Abbildungseigenschaften eines konventionellen Operationsmikroskops vergleichbar.

Die Erfindung wird nachstehend anhand der in den Figuren dargestellten Ausführungsbeispiele näher erläutert.

Es zeigen:

Fig. 1a          eine Schnittdarstellung eines ersten Ausführungsbeispiels der Erfindung zur stereoskopischen Beobachtung durch ein Operationsmikroskop;

Fig. 1b          einen Linsenschnitt des stabförmigen Vorsatzes der in Figur la dargestellten Anordnung;

Fig. 2a          eine zweites Ausführungsbeispiel der Erfindung zur stereoskopischen Abbildung unter Verwendung einer.Stereo-TV-Kamera mit zwei CCD-Bildempfängern;

Fig. 2b          den Linsenschnitt des stabförmigen Vorsatz es aus Figur 2a;

Fig. 3a-3c       eine Prinzipskizze des endoskopischen Vorsatzes zur Erläuterung der Erfindung;

Figur 4          eine vergrößerte Darstellung der stereoskopischen Strahlengänge im Bereich des umgekehrten Fernrohres;

Fig. 5 und 6     Ausführungsbeispiele für die Ausführung der Übertragungsoptik aus identischen und symmetrischen Kittgliedern und

Fig. 7 und 8     die Linsenschnitte zweier weiterer Ausführungsbeispiele der Erfindung.

In Figur la ist der schematische Aufbau eines Operationsmikroskopes, bestehend aus Hauptobjektiv (41), Verqrößerungssystem (42), Tubus (43) und Okular (44) dargestellt. Über ein Anschlußstück (45) ist mit dem Operationsmikroskop ein stabförmiger Vorsatz (46) verbunden, der ein objektseitiges Objektiv (51), ein hinteres Abbildungslinsensystem (50), ein hinteres Feldlinsensystem (49), eine vordere Abbildungsoptik (48) und ein vorderes Feldlinsensystem (47) enthält. Statt der Luftabstände zwischen den Linsensystemen sind Glasstäbe (52) zur Baulängenverlängerung vorgesehen.

Das betrachtete Objekt (53) wird über ein im hinteren Feldlinsensystem (49) entstandenes erstes Zwischenbild (54) als zweites Zwischenbild (55) in die Brennebene des Hauptobjektives (41), die zwischen dem vorderen Feldlinsensystem (47) und dem Hauptobjektiv (41) liegt, abgebildet. Das Hauptobjektiv (41) zusammen mit den beiden Tubuslinsen im Tubus (43) bildet das zweite Zwischenbild (55) als zwei Stereoteilbilder in die Brennebene der Okulare (44) ab. Das Hauptobjektiv (41) bildet zusammen mit der vorderen Abbildungsoptik (48) und der Feldlinse (47) ein umgekehrtes Fernrohr, das eine vignettierungsfreie Einkopplung und Verflechtung der in der Ebene der Gerätepupillen (EP) räumlich getrennten Stereoteilstrahlengänge in den Endoskop-Vorsatz gewährleistet. Die Einkopplung in das Operationsmikroskop erfolgt dabei unter Beibehaltung des Stereowinkels, unter dem das Zwischenbild (55) im Endoskop-Vorsatz erzeugt ist.

In Figur 2a ist das Hauptobjektiv (56) zusätzlich zu den Elementen (47,48,49,50,51), die denen im Ausführungsbeispiel nach Fig. la entsprechen, am betrachtungsseitigen Ausgang des stabförmigen optischen Systems vorgesehen, dem zwei identische Teilobjektive (57.1 und 57.2) für die beiden stereoskopischen Strahlengänge nachgeordnet sind. Den Teilobjektiven (57.1 und 57.2) sind Umlenkprismen (58.1 und 58.2) zur körperlichen Trennung der Stereostrahlengänge sowie CCD-Empfänger (59.1 und 59.2) zur stereoskopischen Aufzeichnung der Teilbilder nachgeordnet.

Das Objektiv (56) ist dem Durchmesser des Rohres angepaßt, so daß sich eine effektive Rohrverlängerung ergibt. Da die Brennweite des Objektives (56) kleiner ist als die des Hauptobjektivs (41) in Figur la, kann in diesem Ausführungsbeispiel auch die Stereobasis proportional der Brennweite verkleinert werden ohne daß der Stereoeindruck dadurch verschlechtert wird.

Mit (EP) ist die Gerätepupille oder die Eintrittspupille des jeweiligen optischen Systems bezeichnet und mit (a) die Stereobasis.

Weiterhin können auch zusätzliche Strahlengänge zur Mitbeobachtung oder Dokumentation vorgesehen sein, die mit den stereoskopischen Strahlengängen für den Hauptbeobachter verflochten sind. Dieses kann beispielsweise einfach durch eine Gestaltung des Operationsmikroskopes oberhalb des Hauptobjektives (41) entsprechend der US-A-4,991,947 erfolgen.

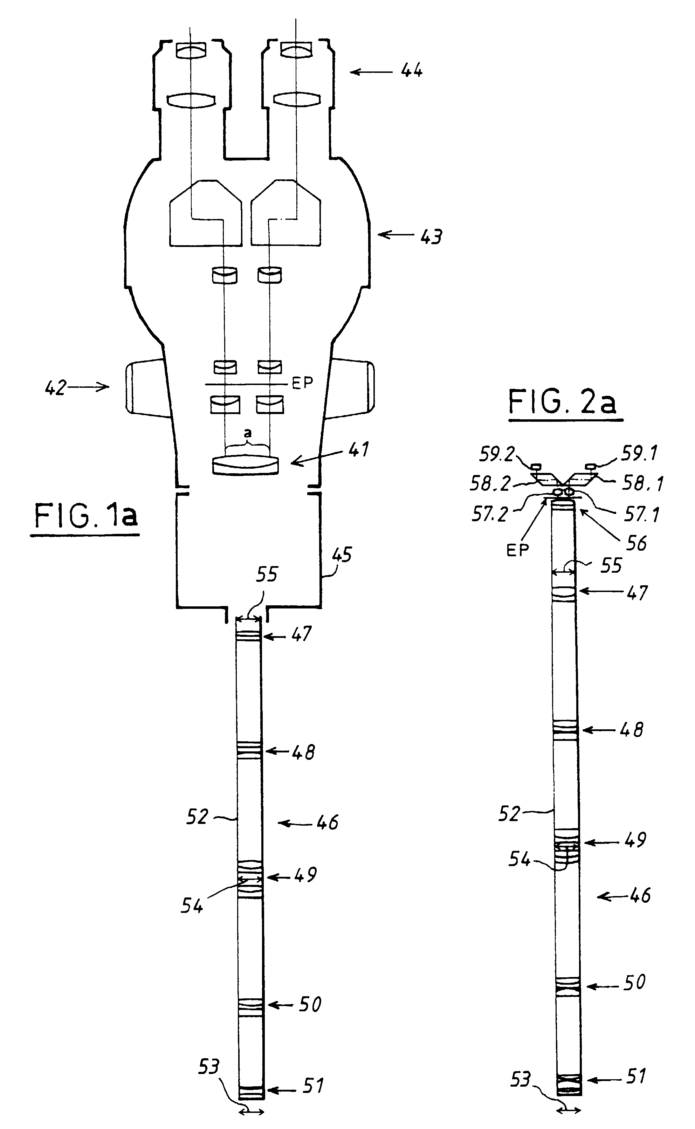

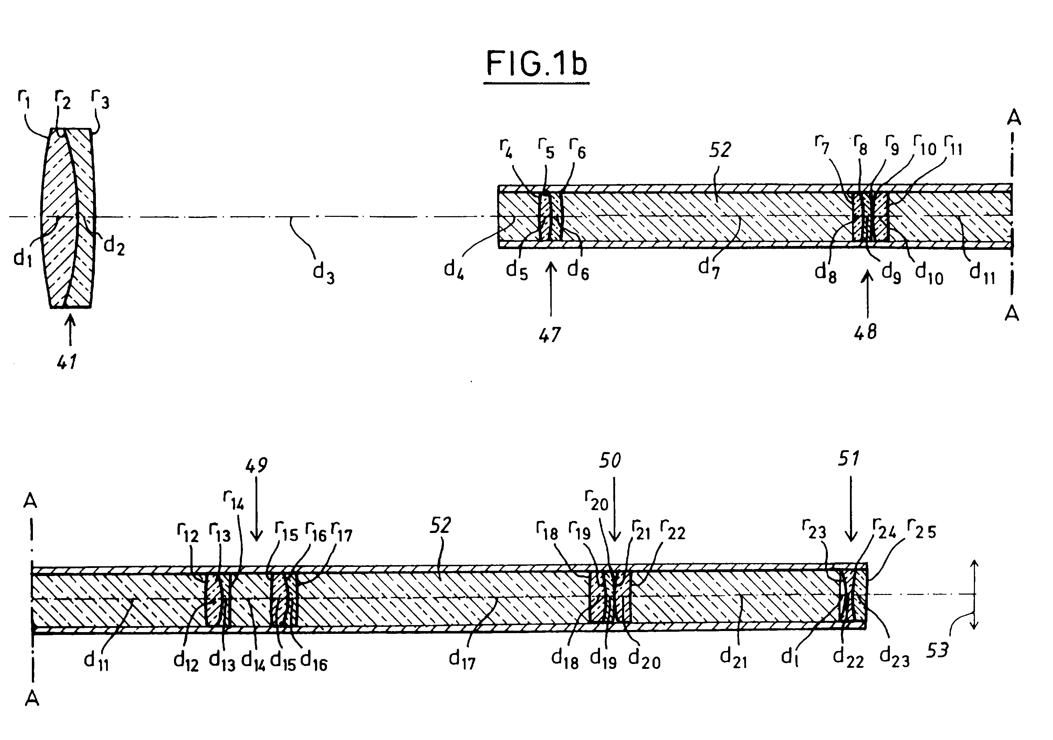

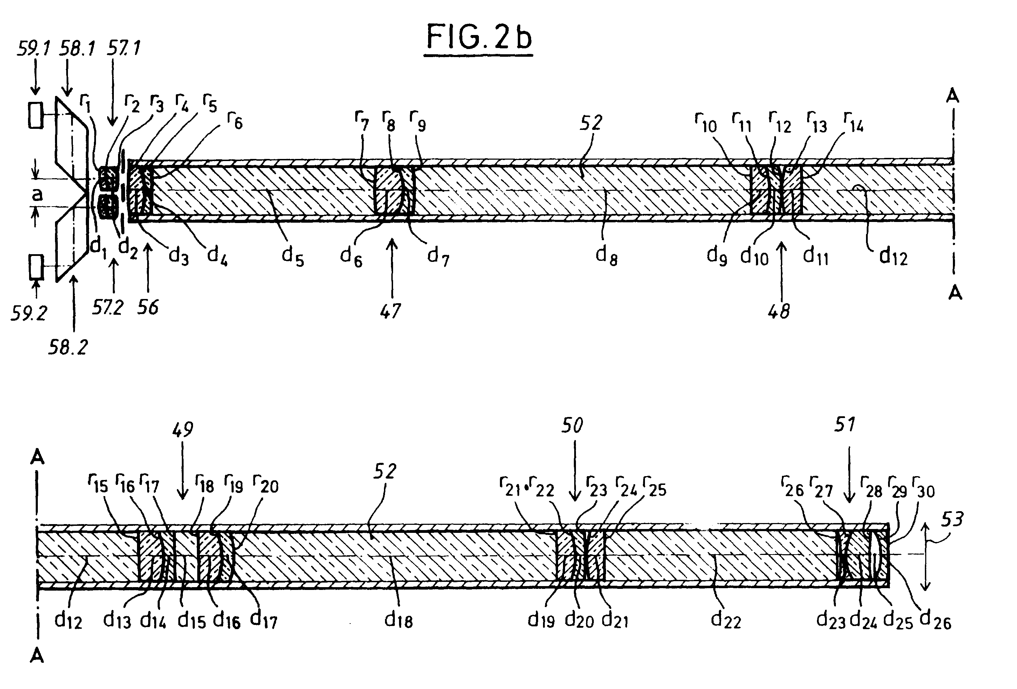

In der Fig. lb ist der optische Aufbau des Endoskopes aus Fig. la zwischen dem objektseitigen Objektiv (51) und dem Hauptobjektiv (41) und in der Fig. 2b das Endoskop aus Fig. 2a vergrößert dargestellt. Die Daten für die Radien r, Dicken und Abstände d sowie die verwendeten Glassorten der optischen Komponenten sind in den nachfolgenden Tabellen I (Fig. 1b) und II (Fig. 2b) angegeben. Mit r ist dabei der jeweilige Flächenkrümmungsradius bezeichnet. Die Dicken und Abstände sind jeweils entlang der strichpunktiert dargestellten optischen Achse zwischen den Schnittpunkten jeweils benachbarter Flächen mit der optischen Achse gemessen. Mit (52) sind Glaszwischenstücke zur Baulängenvergrößerung bezeichnet. Das Endoskop nach Fig. 1b und Tabelle I weist zwischen dem Objektiv (51) und dem sich daran anschließenden Glaszwischenstück (52) einen Luftabstand ($d_1$) von 2 mm auf. Beim Endoskop nach Fig. 2b und Tabelle II ist ein entsprechender Luftabstand ($d_1$) von 1,5 mm vorgesehen.

Statt der dargestellten Anordnungen der optischen Elemente in Figur 1b und 2b, in denen zwei Zwischenbilder (54,55) erzeugt werden, ist auch eine andere geradzahlige Anzahl von Zwischenabbildungen möglich, damit das Objekt höhenund seitenrichtig sowie stereoskopisch richtig betrachtet werden kann. Prinzipiell sind auch ungerade Anzahlen an Zwischenabbildungen möglich, wenn beispielsweise durch Umdrehen der Kameras und links-rechts vertauschte Darbietung die höhen- und seitenrichtige sowie stereoskopisch richtige Darstellung gewährleistet ist. Dabei können auch Teile der offenbarten Linsensysteme (47-51) vorteilhaft mehrfach Verwendung finden. Variationen einzelner Parameter der aufgeführten Datensätze können ebenfalls zu einem vergleichbar guten Abbildungsergebnis führen.

Tabelle I

| Opt.Element | Radius $r_i$/mm | Dicke $d_i$/mm | Abstand $d_i$/mm | Glaswerte |
|---|---|---|---|---|
| 41 | $r_1 = 96.466$ | $d_1 = 8.3$ | | BALF4 |
| | $r_2 = -76.076$ | $d_2 = 4.3$ | | SF54 |
| | $r_3 = -262.7$ | | | |
| | | | $d_3 = 140.5$ | |
| 52 | | | $d_4 = 10.0$ | BK7 |
| | $r_4 = 51.212$ | $d_5 = 3.0$ | | SK11 |
| 47 | $r_5 = -14.343$ | $d_6 = 2.0$ | | SFL6 |
| | $r_6 = -27.582$ | | | |
| 52 | | | $d_7 = 70.0$ | BK7 |
| | $r_7 = 403.88$ | $d_8 = 3.0$ | | SK2 |
| | $r_8 = -15.399$ | | | |
| 48 | $r_9 = -28.592$ | $d_9 = 2.0$ | | SFL6 |
| | $r_{10}= 43.401$ | $d_{10} = 3.0$ | | SSKN8 |
| 52 | $r_{11} = -143.3$ | | $d_{11} = 70.0$ | BK7 |
| | $r_{12}= 31.623$ | $d_{12} = 4.0$ | | SK11 |
| 49 | $r_{13}= -14.962$ | $d_{13} = 2.0$ | | SF10 |
| | $r_{14}= -69.283$ | | | |
| 52 | | | $d_{14} = 10.0$ | BK7 |
| | $r_{15}= 151.79$ | $d_{15} = 4.0$ | | SK11 |
| 49 | $r_{16}= -11.363$ | $d_{16} = 2.0$ | | SFL6 |
| | $r_{17}= -19.953$ | | | |
| 52 | | | $d_{17} = 70.0$ | BK7 |
| | $r_{18}= 316.23$ | | | |
| | | $d_{18} = 4.0$ | | SSKN8 |
| | $r_{19}= -16.079$ | | | |
| 50 | | $d_{19} = 2.0$ | | SFL6 |

Tabelle I   (fortgesetzt)

| Opt.Element | Radius $r_i$/mm | Dicke $d_i$/mm | Abstand $d_i$/mm | Glaswerte |
|---|---|---|---|---|
| | $r_{20}$= -34.974 | | | |
| | $r_{21}$= 29.427 | $d_{20}$ = 3.0 | | SK5 |
| 52 | $r_{22}$= -199.53 | | $d_{21}$ = 50.0 | BK7 |
| | | | $d_1$ = 2.0 | Luft |
| 51 | $r_{23}$= -13.725 | $d_{22}$ = 2.0 | | SK11 |
| | $r_{24}$= 51.955 | $d_{23}$ = 3.0 | | SF10 |
| | $r_{25}$= -104.41 | | | |

Tabelle II

| Opt. Element | Radius $r_i$/mm | Dicke $d_i$/mm | Abstand $d_i$/mm | Glaswerte |
|---|---|---|---|---|
| 57.1,57.2 | $r_1$ = 65.879 | | | |
| | | $d_1$ = 1.0 | | SF10 |
| | $r_2$ = 15.179 | $d_2$ = 1.5 | | SSK50 |
| | $r_3$ = -22.876 | | | |
| 56 | $r_4$ = 21.288 | $d_3$ = 3.0 | | SSKN8 |
| | $r_5$ = -9.8571 | $d_4$ = 1.0 | | SF8 |
| | $r_6$ = -36.256 | | | |
| 52 | | | $d_5$ = 37.2 | BK7 |
| | $r_7$ = 19.248 | $d_6$ = 5.0 | | SK11 |
| 47 | $r_8$ = -5.7876 | $d_7$ = 2.0 | | SFL6 |
| | $r_9$ = -10.981 | | | |
| 52 | | | $d_8$ = 56.83 | SF1 |
| | $r_{10}$= 77.736 | $d_9$ = 3.0 | | SK2 |
| | $r_{11}$= -10.218 | | | |
| 48 | $r_{12}$= -22.227 | $d_{10}$ = 2.0 | | SF10 |
| | $r_{13}$= 28.799 | $d_{11}$ = 3.0 | | SF8 |
| | $r_{14}$= -285.92 | | | |
| 52 | | | $d_{12}$ = 42.99 | SF1 |
| | $r_{15}$= 330.18 | $d_{13}$ = 4.0 | | SSK5I |
| | $r_{16}$= -11.383 | $d_{14}$ = 2.0 | | SF10 |
| | $r_{17}$= -258.52 | $d_{15}$ = 4.0 | | SF1 |
| 49 | $r_{18}$= 196.68 | $d_{16}$ = 4.0 | | SSKN8 |
| | $r_{19}$= -7.1821 | $d_{17}$ = 2.0 | | SF55 |
| | $r_{20}$= -10.0 | | | |
| 52 | | | $d_{18}$ = 54.16 | SF1 |
| | $r_{21}$= 196.68 | $d_{19}$ = 3.0 | | SK2 |
| | $r_{22}$= -9.7163 | | | |
| 50 | $r_{23}$= -24.76 | $d_{20}$ = 2.0 | | SF1 |
| | $r_{24}$= 20.535 | $d_{21}$ = 3.0 | | F5 |
| | $r_{25}$= -149.62 | | | |
| 52 | | | $d_{22}$ = 38.29 | SF1 |
| | | | $d_1$ = 1.5 | Luft |
| | $r_{26}$= -18.701 | $d_{23}$ = 1.0 | | SSK51 |
| | $r_{27}$= 4.5973 | $d_{24}$ = 4.0 | | SF1 |

Tabelle II   (fortgesetzt)

| Opt. Element | Radius $r_i$/mm | Dicke $d_i$/mm | Abstand $d_i$/mm | Glaswerte |
|---|---|---|---|---|
| 51 | $r_{28}$= 38.404 | | | |
| | $r_{29}$= -5.4639 | $d_{25}$ = 2.0 | | Luft |
| | $r_{30}$= -31.623 | $d_{26}$ = 1.0 | | SF10 |

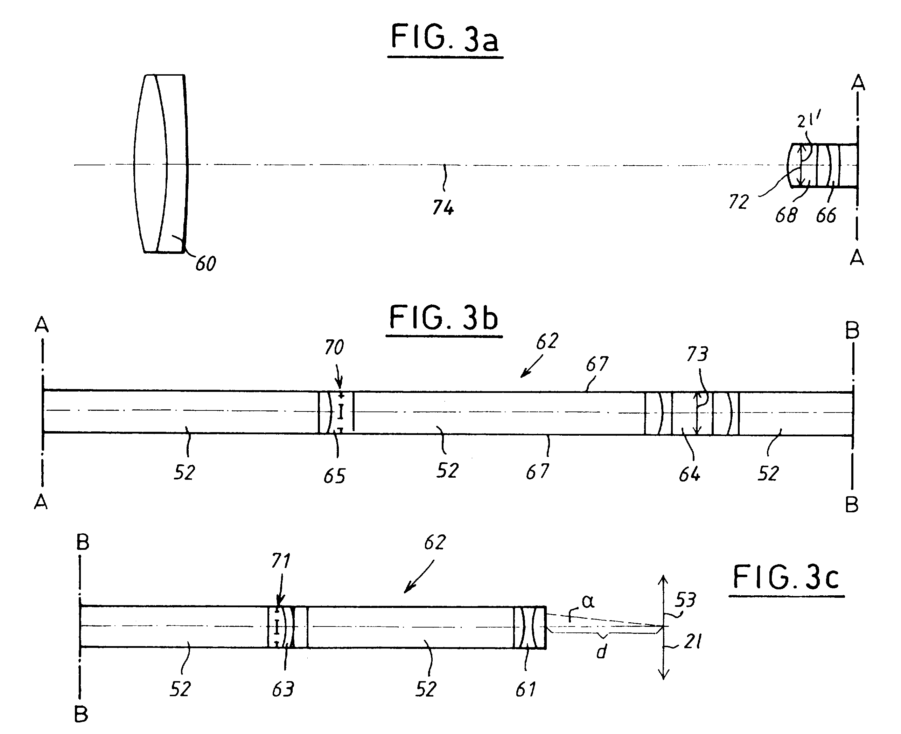

Der in den Figuren 3a bis 3c gezeigte Vorsatz des stereoendoskops besteht aus einem betrachtungsseitigen Objektiv (60) (Hauptobjektiv), einer Einkoppeloptik (66,68), einer Übertragungsoptik (62) und einem objektseitigen Objektiv (61) (Endoskopobjektiv). Die Übertragungsoptik (62) besteht im gezeichneten Ausführungsbeispiel aus drei einzelnen Gliedern (63,64,65) und den Glasstäben (52). Sie kann aber auch aus einer mehrfachen Anzahl dieser Glieder bestehen. Hauptobjektiv (60), Einkoppeloptik (66, 68) und das benachbarte Glied (65) der Übertragungsoptik stellen auch hier ein umgekehrtes Kepler'sches Fernrohr dar. In der Nähe des Zwischenbildes (72) innerhalb des Fernrohres befindet sich noch eine Feldlinse (68), mit der der Hauptstrahlengang (HS) beeinflußt und z.B. im Vorsatz (67) am Ort des Zwischenbildes (72), telezentrisch gemacht werden kann.

Durch das optische System für die stereoskopische Übertragung der Stereostrahlengänge (Übertragungsoptik) (63,64,65) werden sowohl das Zwischenbild (72,73) als auch die Pupillen (70,71) im Maßstab 1:1 übertragen. Diese Übertragung kann auch mit Gradientenstäben erfolgen. Gradientenstäbe haben ein radiales Brechzahlprofil und abbildende (selbstfokussierende) Eigenschaften. Sie werden unter dem Markennamen "SELFOC" angeboten. Ihre bevorzugte Anwendung kann bei kleinen Endoskopröhrendurchmessern von weniger als 3 mm erfolgen.

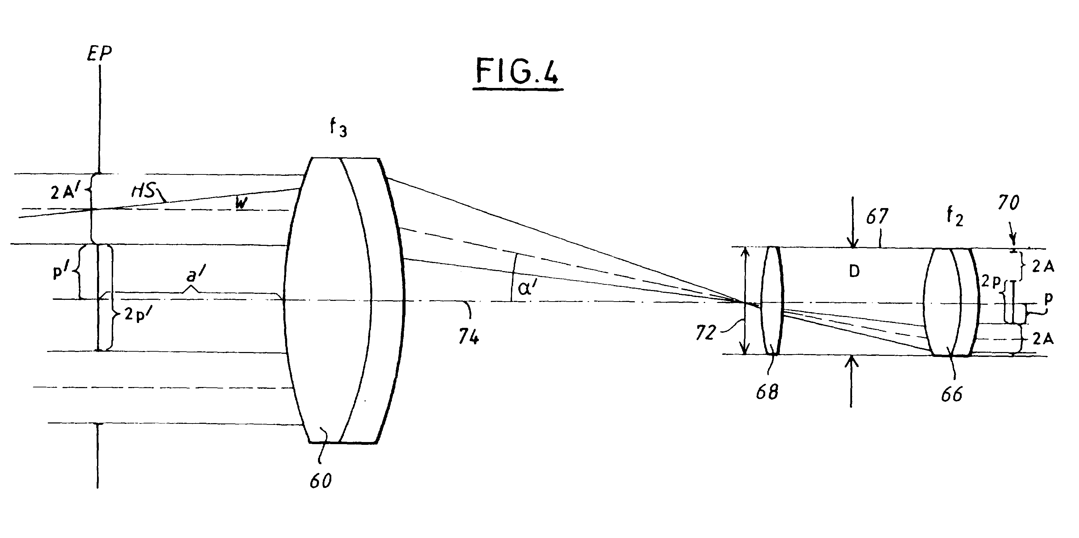

Der in Figur 4 gezeigte Teil des Vorsatz es ist für alle verschiedenen Anwendungsfälle im Prinzip gleich. Schnittstellen sind die anwendungsspezifischen Gerätepupillen (EP) des stereoskopischen Beobachtungssystems, deren Durchmesser (2A'), deren Abstand vom Hauptobjektiv (a') und räumliche Trennung 2 (p'+ A') = Stereobasis je nach Anwendung verschieden sind. Die Gerätepupillen (EP) fallen mit den Bildern der Eintrittspupille (71) des endoskopischen Vorsatzes (Austrittspupille) zusammen.

Der Durchmesser der Gerätepupille oder Austrittspupille (EP) des Endoskops ist mit (2A') und die dazugehörige Stereobasis mit 2 (p'+ A') bezeichnet. Die im Endoskoprohr (67) übertragene Größe der Pupillen trägt jeweils die Bezeichnung (2A), die entsprechende Stereobasis die Bezeichnung 2 (p+A). Mit (D) ist der freie Durchmesser der Optik des Endoskoprohrs bezeichnet und mit (72) das vom Hauptobjektiv (60) betrachtete Zwischenbild. Der Hauptstrahl (HS) der Abbildung bildet mit der zur optischen Achse (74) parallelen Achse den Winkel (w).

Für die Anpassung des Lisensystems im stabförmigen Vorsatz mit einem freien Durchmesser (D) an ein anwendungsspezifisches optisches Gerätesystem sind folgende Bedingungen erfüllt:

$$21' = 2\,f_3 \cdot \tang w = D$$

$$2\,(2A + p) = D$$

und

$$\frac{p}{p'} = \frac{A}{A'} = \frac{f_2}{f_3}$$

Daraus resultiert ein Stereowinkel von

$$\tang \alpha' = \frac{p' + A'}{f_3}$$

Der Abbildungsmaßstab für die Pupillenabbildung ist so gewählt, daß das Bild aus der Summe aus Stereobasis (2(p' + A')) und Pupillendurchmesser (2A') dem freien Durchmesser (D) im Vorsatz entspricht.

Mit dem in den Figuren 3a - 3c dargestellten Übertragungssystem (63,64,65) werden mit einem einzigen zentrischen um die optisch Achse (74) angeordneten optischen System zwei oder auch mehrere stereoskopische Strahlengänge, die paarweise jeweils ein Stereobild liefern, vignettierungsfrei und unter Beibehaltung des Stereoeindrucks und unter Ausnutzung des gesamten freien Optikdurchmessers übertragen. Diese Übertragung geschieht durch mehrmalige Abbildung im Maßstab 1:1 sowohl des Zwischenbildes (72,73) als auch der Pupillen (70,71). Die Anzahl dieser Abbildungen bestimmt bekanntlich Bildorientierung und stereoskopisch richtige oder falsche Tiefenwahrnehmung.

Mit dem Endoskopobjektiv (61) kann vor allem der anwendungsspezifische Objektfelddurchmesser (21) und der Arbeitsabstand (d) festgelegt werden. Für den Abbildungsmaßstab (β) Objekt-Zwischenbild gilt dann:

$$\beta = \frac{1}{1'}$$

Dabei ist mit (21') der Felddurchmesser im Zwischenbild (72) bezeichnet. Mit diesem Abbildungsmaßstab (β) sind aber auch in bekannter Weise die optischen Abbildungseigenschaften wie Auflösung und Tiefenschärfe zwangsweise verknüpft. Der Stereowinkel (α) und damit der stereoeindruck ist umgekehrt proportional zum Abbildungsmaßstab (β):

$$\alpha = \frac{\alpha'}{\beta}$$

Für β = 1 bleibt also der Stereoeindruck unverändert. Für β > 1 erhält man einen verminderten Stereoeindruck, wohingegen die damit zwangsweise zunehmende Tiefenschärfe ein gewünschter Nebeneffekt bei endoskopischen Anwendungen ist.

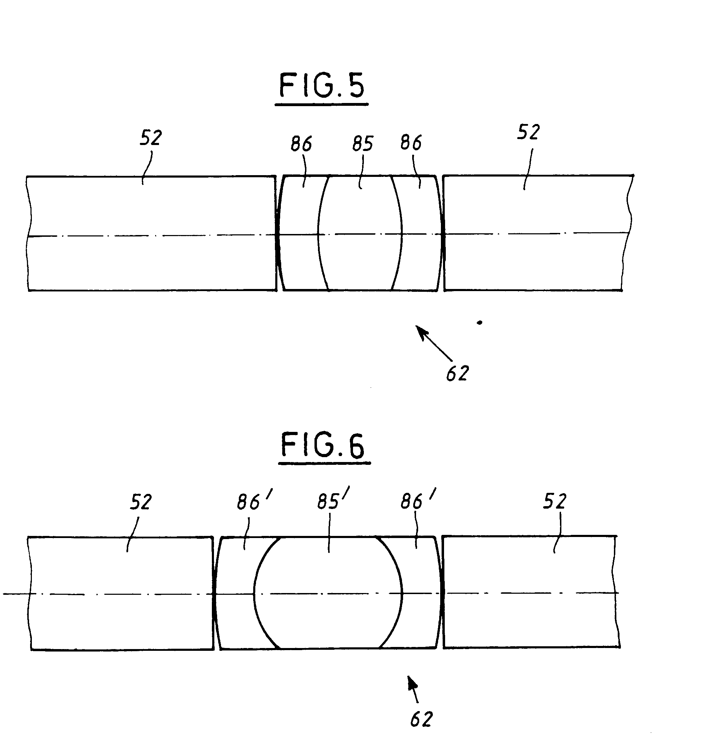

In den in den Figuren 5 und 6 gezeigten Ausführungsbeispielen für die Übertragungsoptik (62) sind die Glasstäbe wieder mit (52) bezeichnet. Die Kittglieder bestehen jeweils aus einer Bikonvexlinse (85, 85'), die zwischen zwei identischen Meniskuslinsen (86, 86') angeordnet sind. Die axiale Länge der Meniskuslinsen (86, 86') ist kleiner als deren halber Durchmesser. Die Glasstäbe (52) schließen sich jeweils unmittelbar, d.h. ohne Luftabstand, an die Meniskuslinsen an. Die Bikonvexlinse (85') in Fig. 6 ist eine Kugellinse.

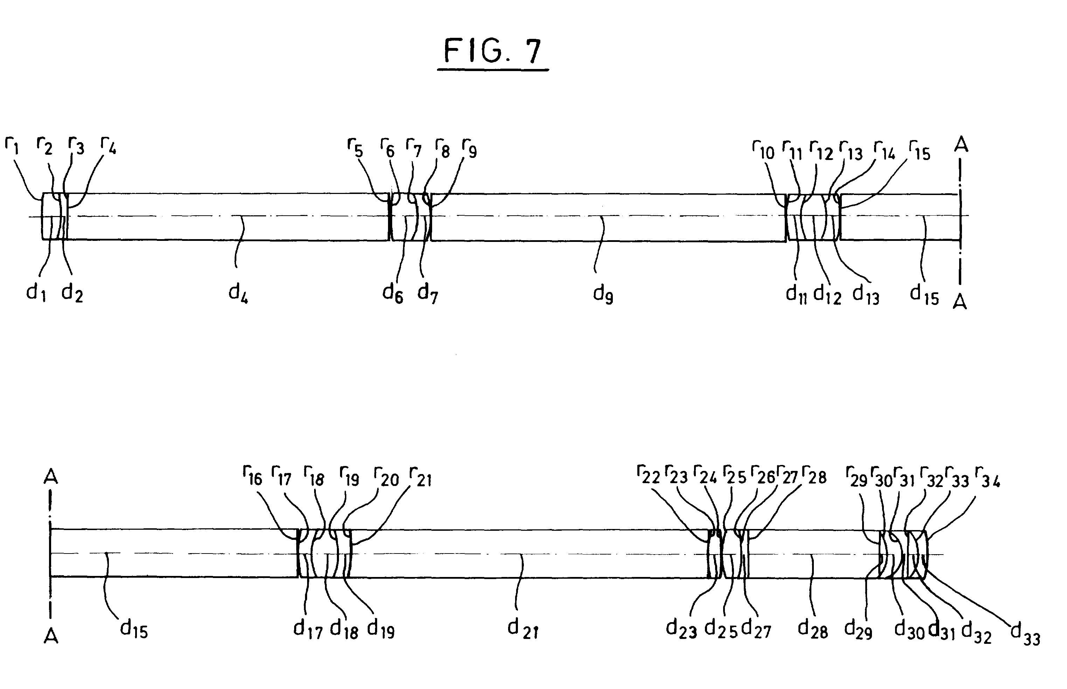

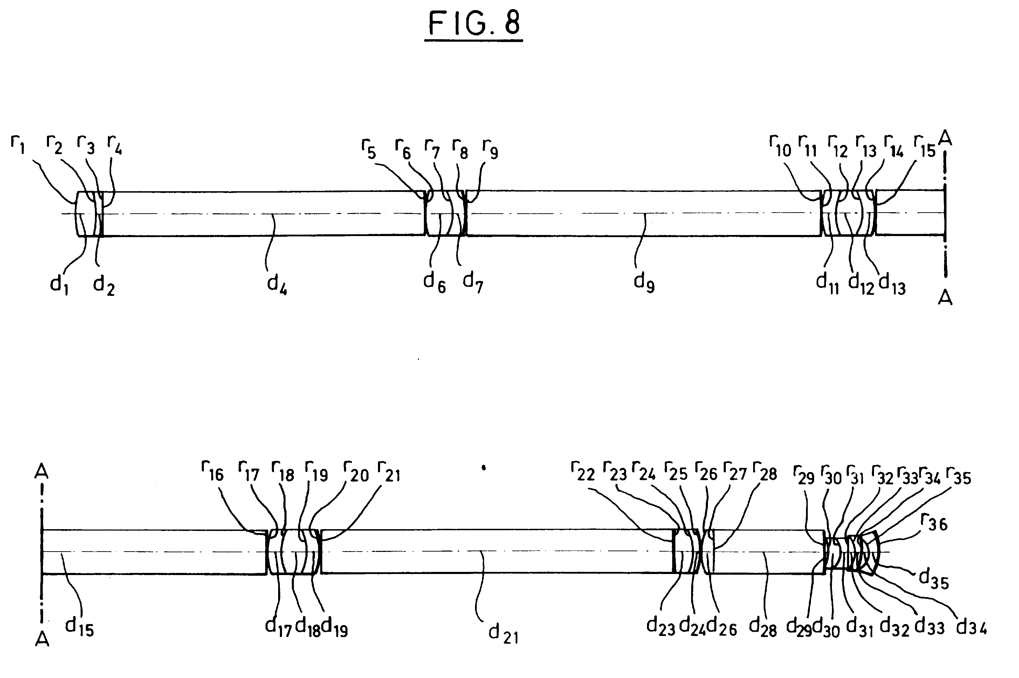

In den Fig. 7 und 8 sind die Linsenschnitte zweier weiterer Ausführungsbeispiele für stabfömige endoskopische Vorsätze dargestellt. Die optischen Daten für die Flächenkrümmungsradien $r_i$, Dicken und Abstände $d_1$ und die verwendeten Gläser des Ausführungsbeispiels nach Fig. 7 sind in Tabelle III und des Ausführungsbeispiels nach Fig. 8 in Tabelle IV angegeben. Die Abstände und Dicken sind dabei entlang der optischen Achse (74) zwischen den Schnittpunkten der jeweiligen Fläche mit der optischen Achse gemessen. Die einzelnen Flächen sind in den Tabelllen III und IV fortlaufend, beim Hauptobjektiv beginnend, nummeriert. Der Endoskopvorsatz in Fig. 7 hat einen Bildwinkel von 45° und der Vorsatz aus Fig. 8 einen Bildwinkel von 60°.

## Tabelle III

| Teilsystem | Nr. | Radius $r_i$/mm | Dicke/Abstand $d_i$/mm | Glaswerte |
|---|---|---|---|---|
| | | | 2.08 | |
| Haupt-objektiv + Einkoppel-optik | 1 | 28.387 | | |
| | | | 3.0 | BaF4 |
| | 2 | -14.855 | | |
| | | | 1.0 | SF56A |
| | 3 | -35.227 | | |
| | | | 0 | |
| | 4 | ∞ | | |
| | | | 48.62 | LAKN22 |
| | 5 | ∞ | | |
| | | | 0 | |
| | 6 | 20.833 | | |
| | | | 4.0 | F5 |
| Über-tragungs-optik | 7 | -7.9433 | | |
| | | | 2.0 | SF56A |
| | 8 | -15.510 | | |
| | | | 0 | |
| | 9 | ∞ | | |
| | | | 53.85 | LAKN22 |
| | 10 | ∞ | | |
| | | | 0 | |
| | 11 | 15.51 | | |
| | | | 2.0 | SF56A |
| | 12 | 7.9433 | | |
| | | | 4.0 | F5 |
| | 13 | -7.9433 | | |
| | | | 2.0 | SF56A |
| | 14 | -15.51 | | |
| | | | 0 | |
| | 15 | ∞ | | |
| | | | 53.85 | LAKN22 |
| | 16 | ∞ | | |
| | | | 0 | |
| | 17 | 15.51 | | |
| | | | 2.0 | SF56A |
| | 18 | 7.9433 | | |
| | | | 4.0 | F5 |
| | 19 | -7.9433 | | |
| | | | 2.0 | SF56A |
| | 20 | -15.51 | | |
| | | | 0 | |
| | 21 | ∞ | | |
| | | | 53.85 | LAKN22 |
| | 22 | ∞ | | |
| | | | 0 | |
| | 23 | 21.288 | | |
| | | | 2.0 | SSKN8 |
| | 24 | -147.49 | | |
| | | | 0 | |

| | | | | |
|---|---|---|---|---|
| | 25 | 12.68 | | |
| | | | 3.0 | SSKN8 |
| | 26 | −15.399 | | |
| | | | 1.0 | SF56A |
| Endoskop-<br>objektiv<br>ß = 6<br>Bild-<br>winkel<br>45° | 27 | −258.52 | | |
| | | | 0 | |
| | 28 | $\infty$ | | |
| | | | 19.79 | SF10 |
| | 29 | $\infty$ | | |
| | | | 1.4 | |
| | 30 | −5.233 | | |
| | | | 2.0 | SF10 |
| | 31 | −3.7584 | | |
| | | | 1.0 | SK11 |
| | 32 | −76.076 | | |
| | | | 1.6 | |
| | 33 | −4.5973 | | |
| | | | 1.4 | BK7 |
| | 34 | −13.626 | | |

Arbeitsabstand d = 45.2 mm, Vergrößerung ß = 6x
Sehfelddurchmesser 2l = 40 mm, Bildwinkel 45°

Tabelle IV

| Teilsystem | Nr. | Radius $r_i$/mm | Dicke/Abstand $d_i$/mm | Glaswerte |
|---|---|---|---|---|
| | | | 2.08 | |
| Haupt-objektiv + Einkoppel-optik | 1 | 28.387 | | |
| | | | 3.0 | BaF4 |
| | 2 | -14.855 | | |
| | | | 1.0 | SF56A |
| | 3 | -35.227 | | |
| | | | 0 | |
| | 4 | ∞ | | |
| | | | 48.62 | LAKN22 |
| | 5 | ∞ | | |
| | | | 0 | |
| | 6 | 20.833 | | |
| | | | 4.0 | F5 |
| Über-tragungs-optik | 7 | -7.9433 | | |
| | | | 2.0 | SF56A |
| | 8 | -15.510 | | |
| | | | 0 | |
| | 9 | ∞ | | |
| | | | 53.85 | LAKN22 |
| | 10 | ∞ | | |
| | | | 0 | |
| | 11 | 15.51 | | |
| | | | 2.0 | SF56A |
| | 12 | 7.9433 | | |
| | | | 4.0 | F5 |
| | 13 | -7.9433 | | |
| | | | 2.0 | SF56A |
| | 14 | -15.51 | | |
| | | | 0 | |
| | 15 | ∞ | | |
| | | | 53.85 | LAKN22 |
| | 16 | ∞ | | |
| | | | 0 | |
| | 17 | 15.51 | | |
| | | | 2.0 | SF56A |
| | 18 | 7.9433 | | |
| | | | 4.0 | F5 |
| | 19 | -7.9433 | | |
| | | | 2.0 | SF56A |
| | 20 | -15.51 | | |
| | | | 0 | |
| | 21 | ∞ | | |
| | | | 53.85 | LAKN22 |
| | 22 | ∞ | | |
| | | | 0 | |
| | 23 | 19.5208 | | |
| | | | 3.0 | SSKN8 |
| | 24 | - 9.60378 | | |

| | | | |
|---|---|---|---|
| 25 | -40.4719 | 1.0 | SF56A |
| | | 0 | |
| 26 | 11.1552 | 2.0 | F5 |
| 27 | -250.0 | 0 | |
| 28 | $\infty$ | 17.11 | SF10 |
| 29 | $\infty$ | 0.5 | |
| 30 | -6.1514 | 2.0 | SF10 |
| 31 | -3.25855 | 1.0 | SK11 |
| 32 | 69.5948 | 1.0 | |
| 33 | -3.37032 | 1.0 | BaF4 |
| 34 | -11.0651 | 1.0 | |
| 35 | -3.79897 | 1.5 | BK7 |
| 36 | -8.59761 | | |

Arbeitsabstand d = 46.0 mm, Vergrößerung ß=8x

Sehfelddurchmesser 2l = 52 mm, Bildwinkel 60°

## Patentansprüche

1. Endoskop bestehend aus einem endoskopischen Vorsatz und einem stereoskopischen Betrachtungssystem (42, 43, 44; 57.1, 57.2, 58.1, 58.2, 59.1, 59.2), wobei

   - das Betrachtungssystem Gerätepupillen (EP) mit einem Pupillendurchmesser (2A') und einer Stereobasis 2 (p' + A') aufweist,
   - der endoskopische Vorsatz (46; 67) ein optisches Linsensystem (47, 48, 49, 50, 51; 61, 63, 64, 65) mit einem freien Durchmesser (D) enthält,
   - beide stereoskopischen Strahlengänge im Vorsatz (46; 67) durch das selbe Linsensystem geführt sind,
   - das Linsensystem des Vorsatzes eine Abbildungsoptik (51, 50, 49, 48; 61, 63, 64, 65, 66) zur Erzeugung eines verkleinerten Zwischenbildes (72) eines Objektes (53) und zur Erzeugung einer Austrittspupille (70) des Vorsatzes umfaßt,
   - ein weiteres Abbildungssystem (47, 41; 60, 66, 68) vorgesehen ist, durch das die Gerätepupillen des Beobachtungssystems derart verkleinert in den Vorsatz (46; 67) abgebildet sind, daß die Summe aus dem Bild (2A) der Gerätepupillen und dem Bild der Stereobasis 2(p + A) dem freien Durchmesser (D) entspricht, wobei das Bild (2A) der Gerätepupillen mit der Austrittspupille (70) des Vorsatzes zusammenfällt, und
   - welches weitere Abbildungssystem ein Hauptobjektiv (41; 60) umfaßt, das das Zwischenbild (55; 72) im wesentlichen nach unendlich abbildet.

2. Endoskop nach Anspruch 1, wobei das Zwischenbild (55; 72) unter einem Stereowinkel ($\alpha'$) erzeugt ist und im Betrachtungssystem (42, 43, 44; 57.1, 57.2, 58.1, 58.2, 59.1, 59.2) Stereohalbbilder unter Beibehaltung des Stereowinkels ($\alpha'$) erzeugt sind.

3. Endoskop nach Anspruche 1 oder 2, wobei die Abbildungsoptik zur Erzeugung eines Zwischenbildes (55; 72) ein Endoskop-Objektiv (51; 61) und ein Übertragungssystem (50, 49, 48; 63, 64, 65) umfassen und wobei das Übertragungssystem ein erstes vom Endoskop-Objektiv (51; 61) erzeugtes Zwischenbild (54; 73) in das Zwischenbild (55; 72) abbildet.

4.  Endoskop nach einem der Ansprüche 1 bis 3, wobei ein Teil der Abbildungsoptik ein Einkopplungssystem (48; 66) umfaßt, das gemeinsam mit dem Hauptobjektiv (41; 60) ein umgekehrtes Fernrohr bildet und das Zwischenbild (72) zwischen dem Hauptobjektiv (41; 60) und dem optischen Einkopplungssystem (48; 66) liegt.

5.  Endoskop nach Anspruch 4, wobei die Einkoppeloptik (66, 68) aus einem Objektiv (66) und einer Feldlinse (68) besteht.

6.  Endoskop nach einem der Ansprüche 1-5, wobei für die Objektabbildung die Bedingung

$$0,5 < \frac{(21')}{D} < 1$$

und für die Pupillenabbildung die Bedingung

$$0,5 < \frac{(2\,(2A + p))}{D} < 1$$

erfüllt ist und wobei gleichzeitig für die Einkopplung in das Hauptobjektiv (41; 60) die Bedingung

$$\frac{(p)}{(p')} = \frac{(A)}{(A')} = \frac{(f_2)}{(f_3)}$$

dem Betrag nach erfüllt ist und wobei 21' der Felddurchmesser des Zwischenbildes (55; 72), $f_2$ die Brennweite des Objektivs der Einkoppeloptik (47; 66) und $f_3$ die Brennweite des Hauptobjektivs (41; 60) ist.

7.  Endoskop nach einem der Ansprüche 1-6, wobei das Hauptobjektiv (56) Bestandteil des stabförmigen Vorsatzes (46) ist und in Betrachtungsrichtung vor dem optischen Übertragungssystem (47, 48, 49, 50, 51) angeordnet ist.

8.  Endoskop nach einem der Ansprüche 1-7, wobei mehrere ineinander verflochtene Strahlengänge durch den Vorsatz (46; 62) geführt sind.

9.  Endoskop nach einem der Ansprüche 1-8, wobei das optische Linsensystem des Vorsatzes ein objektseitiges Endoskop-Objektiv (51; 61), ein hinteres Abbildungssystem (50; 63), ein hinteres Feldlinsensystem (49; 64) und ein vorderes Abbildungssystem (48; 65) enthält.

10. Endoskop nach einem der Ansprüche 1-9, wobei das Hauptobjektiv (41; 60) Teil eines Operationsmikroskopes ist.

11. Endoskop nach einem der Ansprüche 1-10, wobei die Zwischenbilder (54, 55; 72, 73) sich symmetrisch von der optischen Achse (74) über annähernd den freien Durchmesser D des Vorsatz es erstrecken.

12. Endoskop nach einem der Ansprüche 3-11, wobei nach dem optischen Übertragungssystem (63, 64, 65) und der Einkoppeloptik (66, 68) lichtbrechende optische Elemente zur Erweiterung der Stereobasis vorgesehen sind.

13. Endoskop nach einem der Ansprüche 1-12, wobei Glasstäbe (52) zur Baulängenverlängerung angeordnet sind.

14. Endoskop nach einem der Ansprüche 3-13, wobei das Übertragungssystem (63, 64, 65) aus einer Gradientenlinse mit radialem Brechzahlverlauf besteht.

15. Endoskop nach einem der Ansprüche 3-14, wobei das Übertragungssystem (63, 64, 65) aus identischen und jeweils symmetrisch aufgebauten Kittgliedern (86, 85, 86; 86', 85', 86') besteht.

16. Endoskop nach Anspruch 15, wobei zwischen den Kittgliedern Zylinder (52) aus optisch durchsichtigem Material angeordnet sind.

EP 0 582 148 B1

17. Endoskop nach Anspruch 15 oder 16, wobei die Kittglieder aus je einer zwischen zwei Meniskuslinsen (86) angeordneten Bikonvexlinse (85) bestehen.

18. Endoskop nach einem der Ansprüche 15 bis 16, wobei die Kittglieder aus je einer zwischen zwei Meniskuslinsen (86') angeordneten Kugellinse (85') bestehen.

19. Endoskop nach Anspruch 17 oder 18, wobei die axiale Länge der Meniskuslinsen (86, 86') kleiner ist als ihr halber Durchmesser.

20. Endoskop nach einem der Ansprüche 17-19, wobei der Abstand zwischen den Meniskuslinsen (86, 86') und den Zylindern (52) dem Betrag nach Null ist.

21. Endoskop nach einem der Ansprüche 15-20, wobei der thermische Ausdehnungskoeffizient der für die Kittglieder (86, 85; 86', 85') verwendeten Glassorten annähernd gleich ist.

22. Endoskop nach einem der Ansprüche 1-21, wobei für die optischen Elemente gegen Erhitzung und gegen Umwelteinflüsse beständige Gläser verwendet sind.

23. Endoskop nach einem der Ansprüche 1-22, wobei das Beobachtungssystem ein Aufzeichnungssystem (59.1, 59.2) enthält.

24. Endoskop nach einem der Ansprüche 1-23, wobei für einen zusätzlichen Beobachter stereoskopische Kanäle durch das optische System geführt sind.

25. Endoskop nach Anspruch 23, wobei dem Hauptobjektiv (56) Teilobjektive (57.1, 57.2), Prismensysteme (58.1, 58.2) sowie CCD-Empfänger (59.1, 59.2) nachgeordnet sind.

26. Endoskop nach einem der Ansprüche 1-25, gekennzeichnet durch die in den nachfolgenden Tabellen I - IV angegebenen optischen Daten für die Flächenkrümmungsradien $r_i$, Dicken bzw. Abstände $d_i$ und verwendeten Gläser:

Tabelle I

| Opt.Element | Radius $r_i$/mm | Dicke $d_i$/mm | Abstand $d_i$/mm | Glaswerte |
|---|---|---|---|---|
| 41 | $r_1 = 96.466$ | $d_1 = 8.3$ | | BALF4 |
| | $r_2 = -76.076$ | $d_2 = 4.3$ | | SF54 |
| | $r_3 = -262.7$ | | | |
| | | | $d_3 = 140.5$ | |
| 52 | | | $d_4 = 10.0$ | BK7 |
| | $r_4 = 51.212$ | $d_5 = 3.0$ | | SK11 |
| 47 | $r_5 = -14.343$ | $d_6 = 2.0$ | | SFL6 |
| | $r_6 = -27.582$ | | | |
| 52 | | | $d_7 = 70.0$ | BK7 |
| | $r_7 = 403.88$ | $d_8 = 3.0$ | | SK2 |
| | $r_8 = -15.399$ | | | |
| 48 | $r_9 = -28.592$ | $d_9 = 2.0$ | | SFL6 |
| | $r_{10} = 43.401$ | $d_{10} = 3.0$ | | SSKN8 |
| | $r_{11} = -143.3$ | | | |
| 52 | | | $d_{11} = 70.0$ | BK7 |
| | $r_{12} = 31.623$ | $d_{12} = 4.0$ | | SK11 |
| 49 | $r_{13} = -14.962$ | $d_{13} = 2.0$ | | SF10 |
| | $r_{14} = -69.283$ | | | |
| 52 | | | $d_{14} = 10.0$ | BK7 |
| | $r_{15} = 151.79$ | $d_{15} = 4.0$ | | SK11 |
| 49 | $r_{16} = -11.363$ | $d_{16} = 2.0$ | | SFL6 |
| | $r_{17} = -19.953$ | | | |

Tabelle I   (fortgesetzt)

| Opt.Element | Radius $r_i$/mm | Dicke $d_i$/mm | Abstand $d_i$/mm | Glaswerte |
|---|---|---|---|---|
| 52 | | | $d_{17} = 70.0$ | BK7 |
| | $r_{18} = 316.23$ | | | |
| | | $d_{18} = 4.0$ | | SSKN8 |
| | $r_{19} = -16.079$ | | | |
| 50 | | $d_{19} = 2.0$ | | SFL6 |
| | $r_{20} = -34.974$ | | | |
| | $r_{21} = 29.427$ | | | |
| | | $d_{20} = 3.0$ | | SK5 |
| | $r_{22} = -199.53$ | | | |
| 52 | | | $d_{21} = 50.0$ | BK7 |
| | | | $d_1 = 2.0$ | Luft |
| | $r_{23} = -13.725$ | | | |
| 51 | | $d_{22} = 2.0$ | | SK11 |
| | $r_{24} = 51.955$ | | | |
| | | $d_{23} = 3.0$ | | SF10 |
| | $r_{25} = -104.41$ | | | |

Tabelle II

| Opt. Element | Radius $r_i$/mm | Dicke $d_i$/mm | Abstand $d_i$/mm | Glaswerte |
|---|---|---|---|---|
| 57.1, 57.2 | $r_1 = 65.879$ | | | |
| | | $d_1 = 1.0$ | | SF10 |
| | $r_2 = 15.179$ | $d_2 = 1.5$ | | SSK50 |
| | $r_3 = -22.876$ | | | |
| 56 | $r_4 = 21.288$ | $d_3 = 3.0$ | | SSKN8 |
| | $r_5 = -9.8571$ | $d_4 = 1.0$ | | SF8 |
| | $r_6 = -36.256$ | | | |
| 52 | | | $d_5 = 37.2$ | BK7 |
| | $r_7 = 19.248$ | $d_6 = 5.0$ | | SK11 |
| 47 | $r_8 = -5.7876$ | $d_7 = 2.0$ | | SFL6 |
| | $r_9 = -10.981$ | | | |
| 52 | | | $d_8 = 56.83$ | SF1 |
| | $r_{10} = 77.736$ | $d_9 = 3.0$ | | SK2 |
| | $r_{11} = -10.218$ | | | |
| 48 | $r_{12} = -22.227$ | $d_{10} = 2.0$ | | SF10 |
| | $r_{13} = 28.799$ | $d_{11} = 3.0$ | | SF8 |
| | $r_{14} = -285.92$ | | | |
| 52 | | | $d_{12} = 42.99$ | SF1 |
| | $r_{15} = 330.18$ | $d_{13} = 4.0$ | | SSK5I |
| | $r_{16} = -11.383$ | $d_{14} = 2.0$ | | SF10 |
| | $r_{17} = -258.52$ | $d_{15} = 4.0$ | | SF1 |
| 49 | $r_{18} = 196.68$ | $d_{16} = 4.0$ | | SSKN8 |
| | $r_{19} = -7.1821$ | $d_{17} = 2.0$ | | SF55 |
| | $r_{20} = -10.0$ | | | |
| 52 | | | $d_{18} = 54.16$ | SF1 |
| | $r_{21} = 196.68$ | $d_{19} = 3.0$ | | SK2 |
| | $r_{22} = -9.7163$ | | | |
| 50 | $r_{23} = -24.76$ | $d_{20} = 2.0$ | | SF1 |
| | $r_{24} = 20.535$ | $d_{21} = 3.0$ | | F5 |

Tabelle II   (fortgesetzt)

| Opt. Element | Radius $r_i$/mm | Dicke $d_i$/mm | Abstand $d_i$/mm | Glaswerte |
|---|---|---|---|---|
| 52 | $r_{25}$= -149.62 | | $d_{22}$ = 38.29 | SF1 |
| | | | $d_1$ = 1.5.. | Luft |
| | $r_{26}$= -18.701 | $d_{23}$ = 1.0 | | SSK5I |
| | $r_{27}$= 4.5973 | $d_{24}$ = 4.0 | | SF1 |
| 51 | $r_{28}$= 38.404 | | | |
| | $r_{29}$= -5.4639 | $d_{25}$ = 2.0 | | Luft |
| | $r_{30}$= -31.623 | $d_{26}$ = 1.0 | | SF10 |

# Tabelle III

| Teilsystem | Nr. | Radius $r_i$/mm | Dicke/Abstand $d_i$/mm | Glaswerte |
|---|---|---|---|---|
| | | | 2.08 | |
| Haupt-objektiv + Einkoppel-optik | 1 | 28.387 | | |
| | | | 3.0 | BaF4 |
| | 2 | -14.855 | | |
| | | | 1.0 | SF56A |
| | 3 | -35.227 | | |
| | | | 0 | |
| | 4 | ∞ | | |
| | | | 48.62 | LAKN22 |
| | 5 | ∞ | | |
| | | | 0 | |
| | 6 | 20.833 | | |
| | | | 4.0 | F5 |
| Über-tragungs-optik | 7 | -7.9433 | | |
| | | | 2.0 | SF56A |
| | 8 | -15.510 | | |
| | | | 0 | |
| | 9 | ∞ | | |
| | | | 53.85 | LAKN22 |
| | 10 | ∞ | | |
| | | | 0 | |
| | 11 | 15.51 | | |
| | | | 2.0 | SF56A |
| | 12 | 7.9433 | | |
| | | | 4.0 | F5 |
| | 13 | -7.9433 | | |
| | | | 2.0 | SF56A |
| | 14 | -15.51 | | |
| | | | 0 | |
| | 15 | ∞ | | |
| | | | 53.85 | LAKN22 |
| | 16 | ∞ | | |
| | | | 0 | |
| | 17 | 15.51 | | |
| | | | 2.0 | SF56A |
| | 18 | 7.9433 | | |
| | | | 4.0 | F5 |
| | 19 | -7.9433 | | |
| | | | 2.0 | SF56A |
| | 20 | -15.51 | | |
| | | | 0 | |
| | 21 | ∞ | | |
| | | | 53.85 | LAKN22 |
| | 22 | ∞ | | |
| | | | 0 | |
| | 23 | 21.288 | | |
| | | | 2.0 | SSKN8 |
| | 24 | -147.49 | | |
| | | | 0 | |

17

| | | | | |
|---|---|---|---|---|
| 25 | 12.68 | | | |
| | | 3.0 | | SSKN8 |
| 26 | -15.399 | | | |
| | | 1.0 | | SF56A |
| 27 | -258.52 | | | |
| | | 0 | | |
| 28 | $\infty$ | | | |
| | | 19.79 | | SF10 |
| 29 | $\infty$ | | | |
| | | 1.4 | | |
| 30 | -5.233 | | | |
| | | 2.0 | | SF10 |
| 31 | -3.7584 | | | |
| | | 1.0 | | SK11 |
| 32 | -76.076 | | | |
| | | 1.6 | | |
| 33 | -4.5973 | | | |
| | | 1.4 | | BK7 |
| 34 | -13.626 | | | |

Endoskop-objektiv $\beta = 6$ Bild-winkel 45°

Arbeitsabstand d = 45.2 mm, Vergrößerung ß = 6x
Sehfelddurchmesser 2l = 40 mm, Bildwinkel 45°

18

## Tabelle IV

| Teilsystem | Nr. | Radius $r_i$/mm | Dicke/Abstand $d_i$/mm | Glaswerte |
|---|---|---|---|---|
| | | | 2.08 | |
| Haupt-objektiv + Einkoppel-optik | 1 | 28.387 | 3.0 | BaF4 |
| | 2 | -14.855 | 1.0 | SF56A |
| | 3 | -35.227 | 0 | |
| | 4 | $\infty$ | 48.62 | LAKN22 |
| | 5 | $\infty$ | 0 | |
| | 6 | 20.833 | 4.0 | F5 |
| Über-tragungs-optik | 7 | -7.9433 | 2.0 | SF56A |
| | 8 | -15.510 | 0 | |
| | 9 | $\infty$ | 53.85 | LAKN22 |
| | 10 | $\infty$ | 0 | |
| | 11 | 15.51 | 2.0 | SF56A |
| | 12 | 7.9433 | 4.0 | F5 |
| | 13 | -7.9433 | 2.0 | SF56A |
| | 14 | -15.51 | 0 | |
| | 15 | $\infty$ | 53.85 | LAKN22 |
| | 16 | $\infty$ | 0 | |
| | 17 | 15.51 | 2.0 | SF56A |
| | 18 | 7.9433 | 4.0 | F5 |
| | 19 | -7.9433 | 2.0 | SF56A |
| | 20 | -15.51 | 0 | |
| | 21 | $\infty$ | 53.85 | LAKN22 |
| | 22 | $\infty$ | 0 | |
| | 23 | 19.5208 | 3.0 | SSKN8 |
| | 24 | - 9.60378 | | |

| | | 1.0 | SF56A |
|---|---|---|---|
| 25 | −40.4719 | | |
| | | 0 | |
| 26 | 11.1552 | | |
| | | 2.0 | F5 |
| 27 | −250.0 | | |
| | | 0 | |
| 28 | $\infty$ | | |
| | | 17.11 | SF10 |
| 29 | $\infty$ | | |
| | | 0.5 | |
| 30 | −6.1514 | | |
| | | 2.0 | SF10 |
| 31 | −3.25855 | | |
| | | 1.0 | SK11 |
| 32 | 69.5948 | | |
| | | 1.0 | |
| 33 | −3.37032 | | |
| | | 1.0 | BaF4 |
| 34 | −11.0651 | | |
| | | 1.0 | |
| 35 | −3.79897 | | |
| | | 1.5 | BK7 |
| 36 | −8.59761 | | |

Arbeitsabstand d = 46.0 mm, Vergrößerung ß=8x

Sehfelddurchmesser 2l = 52 mm, Bildwinkel 60°

**Claims**

1. Endoscope comprising an endoscopic attachment and a stereoscopic viewing system (42, 43, 44; 57.1, 57.2, 58.1, 58.2, 59.1, 59.2), whereby

   - the viewing system comprises apparatus pupils (EP) having a pupil diameter (2A') and a stereoscopic basis 2(p' + A'),
   - the endoscopic attachment (46; 67) comprises an optical lens system (47, 48, 49, 50, 51; 61, 63, 64, 65) having a clear diameter (D),
   - both stereoscopic beam paths are guided in the attachment (46; 67) by a common lens system,
   - the lens system of the attachment comprising an imaging optic (51, 50, 49, 48; 61, 63, 64, 65, 66) for generating a demagnified intermediate image (72) of an object (53) and for generating an exit pupil (70) of the attachment,
   - a further imaging system (47, 41; 60, 66, 68) is provided by which the apparatus pupils of the viewing system are imaged demagnified into the attachment (46; 67) in a manner that the sum of the image (2A) of the apparatus pupils and the image 2 (p + A) of the stereoscopic basis corresponds to the clear diameter (D), whereby the image (2A) of the apparatus pupils coincides with the exit pupil (70) of the attachment, and
   - which further imaging system comprises a main objective (41; 60) which images the intermediate image (55; 72) substantially to infinity.

2. Endoscope according to claim 1, whereby the intermediate image (55; 72) is generated at a stereo angle (a') and whereby the stereoscopic partial images are generated in the viewing system (42, 43, 44; 57.1, 57.2, 58.1, 58.2, 59.1, 59,2) while maintaining the stereo angle ($\alpha$'),

3. Endoscope of claim 1 or 2, whereby the imaging optic for generating an intermediate image (55; 72) comprises an endoscope objective (51; 61) and a transmission system (50, 49, 48; 63, 64, 65) and whereby the transmission system images a first intermediate image (54; 73), generated by the endoscope objective (51; 61) into the intermediate image (55; 72).

4. Endoscope of one of the claims 1 to 3, whereby a portion of the imaging optic comprises an in-coupling system (48; 66), which conjointly with the main objective (41, 60) defines an inverted telescope, and whereby the intermediate image (72) is lying between said main objective (41, 60) and the in-coupling system (48; 66).

5. Endoscope of claim (4), whereby the in-coupling optic (66; 68) comprises an objective (66) and a field lens (68).

6. Endoscope of one of the claims 1 to 5, whereby the condition

$$0,5 < \frac{(2l')}{D} < 1 \qquad (1)$$

is satisfied for imaging the object, and whereby the condition

$$0,5 < \frac{(2\,(2A + p))}{D} < 1 \qquad (2)$$

is satisfied for imaging the pupils, and whereby concurrently the condition

$$\frac{(p)}{(p')} = \frac{(A)}{(A')} = \frac{(f_2)}{(f_3)} \qquad (3)$$

is satisfied for the absolute values for in-coupling into the main objective (41; 60), and whereby 2l' is the field diameter of the intermediate image (55 ; 72), $f_2$ is the focal length of the objective of the in-coupling optic (47; 66) and $f_3$ is the focal length of the main objective (41, 60).

7. Endoscope of one of the claims 1 to 6, whereby the main objective (56) forms a part of the rod-shaped attachment (46) and is arranged forward of the transmission system (47, 48, 49, 50, 51) in the viewing direction.

8. Endoscope of one of the claims 1 to 7, wherein a plurality of mutually intertwining beam path are guided through said attachment (46; 62).

9. Endoscope of one of the claims 1 to 8, whereby the optical lens system of the attachment includes an objectsided and endoscope objective (51; 61), a rearward imaging system (50; 63), a rearward field lens system (49; 64) and a forward imaging system (48; 65).

10. Endoscope of one of the claims 1 to 9, whereby the main objective (41; 60) forms a part of a surgical microscope.

11. Endoscope of one of the claims 1 to 10, whereby the intermediate images (54, 55; 72, 73) extend symmetrically with respect to the optical axis (74) over approximately all of the clear diameter (D).

12. Endoscope of one of the claims 3 to 11, whereby light refracting optical elements for expanding the stereoscopic basis are provided downstream of the optical transmission system (63, 64, 65) and the in-coupling optic (66, 68).

13. Endoscope of one of the claims 1 to 12, whereby glass rods (52) are arranged for extending the structural lengths.

14. Endoscope of one of the claims 3 to 13, whereby the transmission system (63, 64, 65) comprises a gradient lens having a radially dependent refractive index.

15. Endoscope of one of the claims 3 to 14, whereby the transmission system (63, 64, 65) consists of identical cemented optical elements (86, 85, 86; 86', 85', 86') each of which being assembled symmetrically.

16. Endoscope of claim 15, whereby cylinders (52) being made of optically transparent material are arranged between the cemented optical elements.

17. Endoscope of claim 15 or 16, whereby each of the cemented optical elements consists of one bi-convex lens (85) arranged between two meniscus lenses (86).

18. Endoscope of one of the claims 15 to 16, whereby each of the cemented optical elements consists of a spherical lens (85') arranged between two meniscus lenses (86').

19. Endoscope of claim 17 or 18, whereby the axial length of the meniscus lenses (86, 86') is less than one half of its diameter.

20. Endoscope of one of the claims 17 to 19, whereby the distance between the meniscus lenses (86, 86') and the cylinders (52) is zero.

21. Endoscope of one of the claims 15 to 20, whereby the coefficient for thermal expansion of the glass materials used for the cemented elements (86, 85; 86', 85') is approximately the same.

22. Endoscope of one of the claims 1 to 21, whereby for the optical elements glass materials are used which are resistant to heat and environmental influences.

23. Endoscope of one of the claims 1 to 22, whereby the viewing system comprises a recording system (59.1, 59,2).

24. Endoscope of one of the claims 1 to 23, whereby stereoscopic viewing channels for an additional viewer are guided through the optical system.

25. Endoscope of claim 23, whereby partial objectives (57.1, 57.2), prism systems (58,1, 58.2) and CCD-receivers (59.1, 59,2) are arranged downstream of the main objective (56).

26. Endoscope of one of the claims 1 to 25, characterized by the optical data for the radii $r_i$ of surface curvature, thicknesses and distances $d_i$ and used glass materials delineated in one of the following tables I to IV.

Table I

| opt.element | radius $r_i$/mm | thickness $d_i$/mm | distance $d_i$/mm | glass material |
|---|---|---|---|---|
| 41 | $r_1 = 96.466$ | $d_1 = 8.3$ | | BALF4 |
| | $r_2 = -76.076$ | $d_2 = 4.3$ | | SF54 |
| | $r_3 = -262.7$ | | | |
| | | | $d_3 = 140.5$ | |
| 52 | | | $d_4 = 10.0$ | BK7 |
| | $r_4 = 51.212$ | $d_5 = 3.0$ | | SK11 |
| 47 | $r_5 = -14.343$ | $d_6 = 2.0$ | | SFL6 |
| | $r_6 = -27.582$ | | | |
| 52 | | | $d_7 = 70.0$ | BK7 |
| | $r_7 = 403.88$ | $d_8 = 3.0$ | | SK2 |
| | $r_8 = -15.399$ | | | |
| 48 | $r_9 = -28.592$ | $d_9 = 2.0$ | | SFL6 |
| | $r_{10}= 43.401$ | $d_{10} = 3.0$ | | SSKN8 |
| | $r_{11}= -143.3$ | | | |
| 52 | | | $d_{11} = 70.0$ | BK7 |
| | $r_{12}= 31.623$ | $d_{12} = 4.0$ | | SK11 |
| 49 | $r_{13}= -14.962$ | $d_{13} = 2.0$ | | SF10 |
| | $r_{14}= -69.283$ | | | |
| 52 | | | $d_{14} = 10.0$ | BK7 |
| | $r_{15}= 151.79$ | $d_{15} = 4.0$ | | SK11 |
| 49 | $r_{16}= -11.363$ | $d_{16} = 2.0$ | | SFL6 |
| | $r_{17}= -19.953$ | | | |
| 52 | | | $d_{17} = 70.0$ | BK7 |
| | $r_{18}= 316.23$ | | | |

Table I   (continued)

| opt.element | radius $r_i$/mm | thickness $d_i$/mm | distance $d_i$/mm | glass material |
|---|---|---|---|---|
| | | $d_{18}$ = 4.0 | | SSKN8 |
| | $r_{19}$= -16.079 | | | |
| 50 | | $d_{19}$ = 2.0 | | SFL6 |
| | $r_{20}$= -34.974 | | | |
| | $r_{21}$= 29.427 | | | |
| | | $d_{20}$ = 3.0 | | SK5 |
| | $r_{22}$= -199.53 | | | |
| 52 | | | $d_{21}$ = 50.0 | BK7 |
| | | | $d_1$ = 2.0 | Luft |
| | $r_{23}$= -13.725 | | | |
| 51 | | $d_{22}$ = 2.0 | | SK11 |
| | $r_{24}$= 51.955 | | | |
| | | $d_{23}$ = 3.0 | | SF10 |
| | $r_{25}$= -104.41 | | | |

Table II

| opt.element | radius $r_i$/mm | thickness $d_i$/mm | distance $d_i$/mm | glass material |
|---|---|---|---|---|
| 57.1,57.2 | $r_1$ = 65.879 | | | |
| | | $d_1$ = 1.0 | | SF10 |
| | $r_2$ = 15.179 | $d_2$ = 1.5 | | SSK50 |
| | $r_3$ = -22.876 | | | |
| 56 | $r_4$ = 21.288 | $d_3$ = 3.0 | | SSKN8 |
| | $r_5$ = -9.8571 | $d_4$ = 1.0 | | SF8 |
| | $r_6$ = -36.256 | | | |
| 52 | | | $d_5$ = 37.2 | BK7 |
| | $r_7$ = 19.248 | $d_6$ = 5.0 | | SK11 |
| 47 | $r_8$ = -5.7876 | $d_7$ = 2.0 | | SFL6 |
| | $r_9$ = -10.981 | | | |
| 52 | | | $d_8$ = 56.83 | SF1 |
| | $r_{10}$= 77.736 | $d_9$ = 3.0 | | SK2 |
| | $r_{11}$= -10.218 | | | |
| 48 | $r_{12}$= -22.227 | $d_{10}$ = 2.0 | | SF10 |
| | $r_{13}$= 28.799 | $d_{11}$ = 3.0 | | SF8 |
| | $r_{14}$= -285.92 | | | |
| 52 | | | $d_{12}$ = 42.99 | SF1 |
| | $r_{15}$= 330.18 | $d_{13}$ = 4.0 | | SSK5l |
| | $r_{16}$= -11.383 | $d_{14}$ = 2.0 | | SF10 |
| | $r_{17}$= -258.52 | $d_{15}$ = 4.0 | | SF1 |
| 49 | $r_{18}$= 196.68 | $d_{16}$ = 4.0 | | SSKN8 |
| | $r_{19}$= -7.1821 | $d_{17}$ = 2.0 | | SF55 |
| | $r_{20}$= -10.0 | | | |
| 52 | | | $d_{18}$ = 54.16 | SF1 |
| | $r_{21}$= 196.68 | $d_{19}$ = 3.0 | | SK2 |
| | $r_{22}$= -9.7163 | | | |
| 50 | $r_{23}$= -24.76 | $d_{20}$ = 2.0 | | SF1 |
| | $r_{24}$= 20.535 | $d_{21}$= 3.0 | | F5 |
| | $r_{25}$= -149.62 | | | |
| 52 | | | $d_{22}$ = 38.29 | SF1 |

Table II   (continued)

| opt.element | radius $r_i$/mm | thickness $d_i$/mm | distance $d_i$/mm | glass material |
|---|---|---|---|---|
| | | | $d_1 = 1.5..$ | Luft |
| | $r_{26}= -18.701$ | $d_{23} = 1.0$ | | SSK5I |
| | $r_{27}= 4.5973$ | $d_{24} = 4.0$ | | SF1 |
| 51 | $r_{28}= 38.404$ | | | |
| | $r_{29}= -5.4639$ | $d_{25} = 2.0$ | | Luft |
| | $r_{30}= -31.623$ | $d_{26} = 1.0$ | | SF10 |

## Table III

| partial system No. | | radius $r_i$/mm | thickness/distance $d_i$/mm | glass material |
|---|---|---|---|---|
| | | | 2.08 | |
| main objective + in-coupling optic | 1 | 28.387 | | |
| | | | 3.0 | BaF4 |
| | 2 | -14.855 | | |
| | | | 1.0 | SF56A |
| | 3 | -35.227 | | |
| | | | 0 | |
| | 4 | ∞ | | |
| | | | 48.62 | LAKN22 |
| | 5 | ∞ | | |
| | | | 0 | |
| | 6 | 20.833 | | |
| | | | 4.0 | F5 |
| transmission optic | 7 | -7.9433 | | |
| | | | 2.0 | SF56A |
| | 8 | -15.510 | | |
| | | | 0 | |
| | 9 | ∞ | | |
| | | | 53.85 | LAKN22 |
| | 10 | ∞ | | |
| | | | 0 | |
| | 11 | 15.51 | | |
| | | | 2.0 | SF56A |
| | 12 | 7.9433 | | |
| | | | 4.0 | F5 |
| | 13 | -7.9433 | | |
| | | | 2.0 | SF56A |
| | 14 | -15.51 | | |
| | | | 0 | |
| | 15 | ∞ | | |
| | | | 53.85 | LAKN22 |
| | 16 | ∞ | | |
| | | | 0 | |
| | 17 | 15.51 | | |
| | | | 2.0 | SF56A |
| | 18 | 7.9433 | | |
| | | | 4.0 | F5 |
| | 19 | -7.9433 | | |
| | | | 2.0 | SF56A |
| | 20 | -15.51 | | |
| | | | 0 | |
| | 21 | ∞ | | |
| | | | 53.85 | LAKN22 |
| | 22 | ∞ | | |
| | | | 0 | |
| | 23 | 21.288 | | |
| | | | 2.0 | SSKN8 |
| | 24 | -147.49 | | |
| | | | 0 | |
| | 25 | 12.68 | | |

| No. | Radius | Thickness | Glass |
|---|---|---|---|
| 26 | -15.399 | 3.0 | SSKN8 |
| | | 1.0 | SF56A |
| 27 | -258.52 | | |
| | | 0 | |
| 28 | ∞ | | |
| 29 | ∞ | 19.79 | SF10 |
| | | 1.4 | |
| 30 | -5.233 | | |
| | | 2.0 | SF10 |
| 31 | -3.7584 | | |
| | | 1.0 | SK11 |
| 32 | -76.076 | | |
| | | 1.6 | |
| 33 | -4.5973 | | |
| | | 1.4 | BK7 |
| 34 | -13.626 | | |

endoscope objective ß = 6 viewing field angle 45°

free working distance d = 45,2mm, magnification ß = 6x
diameter of field of view 21 = 40mm, angle of field of view 45°

# Table IV

| partial system No. | | radius $r_i$/mm | thickness/distance $d_i$/mm | glass material |
|---|---|---|---|---|
| | | | 2.08 | |
| main objective + in-coupling optic | 1 | 28.387 | | |
| | | | 3.0 | BaF4 |
| | 2 | -14.855 | | |
| | | | 1.0 | SF56A |
| | 3 | -35.227 | | |
| | | | 0 | |
| | 4 | ∞ | | |
| | | | 48.62 | LAKN22 |
| | 5 | ∞ | | |
| | | | 0 | |
| | 6 | 20.833 | | |
| transmission optic | | | 4.0 | F5 |
| | 7 | -7.9433 | | |
| | | | 2.0 | SF56A |
| | 8 | -15.510 | | |
| | | | 0 | |
| | 9 | ∞ | | |
| | | | 53.85 | LAKN22 |
| | 10 | ∞ | | |
| | | | 0 | |
| | 11 | 15.51 | | |
| | | | 2.0 | SF56A |
| | 12 | 7.9433 | | |
| | | | 4.0 | F5 |
| | 13 | -7.9433 | | |
| | | | 2.0 | SF56A |
| | 14 | -15.51 | | |
| | | | 0 | |
| | 15 | ∞ | | |
| | | | 53.85 | LAKN22 |
| | 16 | ∞ | | |
| | | | 0 | |
| | 17 | 15.51 | | |
| | | | 2.0 | SF56A |
| | 18 | 7.9433 | | |
| | | | 4.0 | F5 |
| | 19 | -7.9433 | | |
| | | | 2.0 | SF56A |
| | 20 | -15.51 | | |
| | | | 0 | |
| | 21 | ∞ | | |
| | | | 53.85 | LAKN22 |
| | 22 | ∞ | | |
| | | | 0 | |
| | 23 | 19.5208 | | |
| | | | 3.0 | SSKN8 |
| | 24 | - 9.60378 | | |

| | | 1.0 | SF56A |
|----|----------|-------|-------|
| 25 | -40.4719 | | |
| | | 0 | |
| 26 | 11.1552 | 2.0 | F5 |
| 27 | -250.0 | 0 | |
| 28 | $\infty$ | 17.11 | SF10 |
| 29 | $\infty$ | 0.5 | |
| 30 | -6.1514 | 2.0 | SF10 |
| 31 | -3.25855 | 1.0 | SK11 |
| 32 | 69.5948 | 1.0 | |
| 33 | -3.37032 | 1.0 | BaF4 |
| 34 | -11.0651 | 1.0 | |
| 35 | -3.79897 | 1.5 | BK7 |
| 36 | -8.59761 | | |

```
free working distance d = 46,0mm, magnification ß = 8x
diameter of field of view 21 = 52mm, angle of field of view 60°
```

Revendications

1. Endoscope composé d'un dispositif endoscopique additionnel et d'un système d'observation stéréoscopique (42, 43, 44; 57.1, 57.2, 58.1, 58.2, 2, 59.1, 59.2), où

- le système d'observation présente des pupilles (EP) d'un diamètre (2A') et une base stéréoscopique 2(p' + A'),
- le dispositif endoscopique additionnel (46; 67) comprend un système optique de lentilles (47, 48, 49, 50, 51; 61, 63, 64, 65) d'un diamètre utile (D),
- les deux trajets lumineux stéréoscopiques du dispositif endoscopique additionnel (46; 67) traversent le même système de lentilles,
- le système de lentilles du dispositif endoscopique additionnel comprend une optique de reproduction (51, 50, 49, 48; 61, 63, 64, 65, 66) destinée à former une image intermédiaire réduite (72) d'un objet (53) et à former une pupille de sortie (70) du dispositif endoscopique additionnel,
- un autre système de reproduction (47, 41; 60, 66, 68) est prévu qui reproduit les pupilles du système d'observation dans le dispositif endoscopique additionnel (46; 67), en le réduisant, de manière à ce que la somme de l'image (2A) desdites pupilles et de l'image de la base stéréoscopique 2(p' + A') corresponde au diamètre utile (D), l'image (2A) desdites pupilles coïncidant avec la pupille de sortie (70) du dispositif endoscopique additionnel,
- ledit autre système d'observation comprenant un objectif principal (41; 60) qui reproduit l'image intermédiaire (55; 72) sensiblement à l'infini.

2. Endoscope selon la revendication 1, où l'image intermédiaire (55 ; 72) est formée sous un angle stéréoscopique (α') et où le système d'observation (42, 43, 44; 57.1, 57.2, 58.1, 58.2, 2, 59.1, 59.2) forme deux images stéréoscopiques en maintenant l'angle stéréoscopique ($\alpha'$).

3. Endoscope selon la revendication 1 ou 2, où l'optique de reproduction servant à former une image intermédiaire (55; 72) comprend un objectif d'endoscope (51; 61) et un système de transmission (50, 49, 48; 63, 64, 65) et où ledit système de transmission reproduit une première image intermédiaire (54; 73) formée par l'objectif d'endoscope (51; 61) dans l'image intermédiaire (55; 72).

4. Endoscope selon l'une des revendications 1 à 3, où une partie de l'optique de reproduction comprend un système optique d'injection (48; 66) qui forme avec l'objectif principal (41; 60) une lunette inversée et où l'image intermédiaire (72) se trouve entre l'objectif principal (41; 60) et le système optique d'injection (48; 66).

5. Endoscope selon la revendication 4, où l'optique d'injection (66, 68) est composée d'un objectif (66) et d'une lentille de champ (68).

6. Endoscope selon l'une des revendications 1 à 5, où la condition

$$0,5 < \frac{(2l')}{D} < 1 \tag{1}$$

est remplie pour la reproduction de l'objet, où la condition

$$0,5 < \frac{(2\,(2A + p))}{D} < 1 \tag{2}$$

est remplie pour la reproduction des pupilles dans les pupilles de sortie, où la condition

$$\frac{(p)}{(p')} = \frac{(A)}{(A')} = \frac{(f_2)}{(f_3)} \tag{3}$$

est remplie en même temps en valeur absolue pour l'injection optique dans l'objectif principal (41; 60) et où 2l' est le diamètre de champ de l'image intermédiaire (55 ; 72), $f_2$ la focale de l'objectif de l'optique d'injection (47; 66) et $f_3$ la focale de l'objectif principal (41; 60).

7. Endoscope selon l'une des revendications 1 à 6, où l'objectif principal (56) fait partie intégrante du dispositif additionnel en forme de tige (46) et se trouve devant le système optique de transmission (47, 48, 49, 50, 51) dans le sens de l'observation.

8. Endoscope selon l'une des revendications 1 à 7, où plusieurs trajets lumineux entrelacés traversent le dispositif additionnel (46; 62).

9. Endoscope selon l'une des revendications 1 à 8, où le système optique de lentilles du dispositif additionnel comprend un objectif d'endoscope côté objet (51; 61), un système de reproduction postérieur (50; 63), un système de lentilles de champ postérieur (49; 64) et un système de reproduction antérieur (48; 65).

10. Endoscope selon l'une des revendications 1 à 9, où l'objectif principal (41; 60) fait partie intégrante d'un microscope d'opération.

11. Endoscope selon l'une des revendications 1 à 10, où les images intermédiaires (54, 55; 72, 73) s'étendent symétriquement autour de l'axe optique (74) sur presque tout le diamètre utile D du dispositif additionnel.

12. Endoscope selon l'une des revendications 3 à 11, où des éléments optiques réfringents destinés à élargir la base stéréoscopique sont prévus en aval du système optique de transmission (63, 64, 65) et de l'optique d'injection (66, 68).

13. Endoscope selon l'une des revendications 1 à 12, où des tiges de verre (52) sont disposées à titre de rallonge.

14. Endoscope selon l'une des revendications 3 à 13, où le système de transmission (63, 64, 65) est constitué par une lentille à gradient d'indice présentant un profil d'indices radial.

15. Endoscope selon l'une des revendications 3 à 14, où le système de transmission (63, 64, 65) est composé d'éléments cimentés identiques de composition symétrique (86, 85, 86; 86', 85', 86').

16. Endoscope selon la revendication 15, où des cylindres (52) formés par un matériau optiquement transparent sont disposés entre les éléments cimentés.

17. Endoscope selon la revendication 15 ou 16, où les éléments cimentés sont composés respectivement d'une lentille biconvexe (85) disposée entre deux lentilles ménisques (86).

18. Endoscope selon l'une des revendications 15 à 16, où les éléments cimentés sont composés respectivement d'une lentille sphérique (85') disposée entre deux lentilles ménisques (86').

19. Endoscope selon la revendication 17 ou 18, où la longueur axiale des lentilles ménisques (86, 86') est inférieure à la moitié de leur diamètre.

20. Endoscope selon l'une des revendications 17 à 19, où la distance séparant les lentilles ménisques (86, 86') des cylindres (52) est zéro.

21. Endoscope selon l'une des revendications 15 à 20, où le coefficient de dilatation thermique des types de verre utilisés pour la fabrication des éléments cimentés (86, 85; 86', 85') est sensiblement égal.

22. Endoscope selon l'une des revendications 1 à 21, où les éléments optiques sont fabriqués sur la base de verres résistants aux températures élevées et à l'action de l'environnement.

23. Endoscope selon l'une des revendications 1 à 22, où le système d'observation renferme un système d'enregistrement (59.1, 59.2).

24. Endoscope selon l'une des revendications 1 à 23, où des voies stéréoscopiques destinées à un coobservateur traversent le système optique.

25. Endoscope selon la revendication 23, où l'objectif principal (56) est suivi d'objectifs partiels (57.1, 57.2), de systèmes de prismes (58.1, 58.2) et de capteurs CCD (59.1, 59.2).

26. Endoscope selon l'une des revendications 1 à 25, caractérisé par les données optiques relatives aux rayons de courbure $r_i$, aux épaisseurs, aux distances $d_i$ et aux verres utilisés, indiquées dans les tables I - IV ci-après.

Table I

| Elément optique | Rayon $r_i$/mm | Epaisseur $d_i$/mm | Distance $d_i$/mm | Type de verre |
|---|---|---|---|---|
| 41 | $r_1 = 96.466$ | $d_1 = 8.3$ | | BALF4 |
| | $r_2 = -76.076$ | $d_2 = 4.3$ | | SF54 |
| | $r_3 = -262.7$ | | | |
| 52 | | | $d_3 = 140.5$ | |
| | | | $d_4 = 10.0$ | BK7 |
| | $r_4 = 51.212$ | $d_5 = 3.0$ | | SK11 |
| 47 | $r_5 = -14.343$ | $d_6 = 2.0$ | | SFL6 |
| | $r_6 = -27.582$ | | | |
| 52 | | | $d_7 = 70.0$ | BK7 |
| | $r_7 = 403.88$ | $d_8 = 3.0$ | | SK2 |
| | $r_8 = -15.399$ | | | |
| 48 | $r_9 = -28.592$ | $d_9 = 2.0$ | | SFL6 |
| | $r_{10}= 43.401$ | $d_{10} = 3.0$ | | SSKN8 |
| | $r_{11}= -143.3$ | | | |
| 52 | | | $d_{11} = 70.0$ | BK7 |

Table I   (suite)

| Elément optique | Rayon $r_i$/mm | Epaisseur $d_i$/mm | Distance $d_i$/mm | Type de verre |
|---|---|---|---|---|
|  | $r_{12}$= 31.623 | $d_{12}$ = 4.0 |  | SK11 |
| 49 | $r_{13}$= -14.962 | $d_{13}$ = 2.0 |  | SF10 |
|  | $r_{14}$= -69.283 |  |  |  |
| 52 |  |  | $d_{14}$ = 10.0 | BK7 |
|  | $r_{15}$= 151.79 | $d_{15}$ = 4.0 |  | SK11 |
| 49 | $r_{16}$= -11.363 | $d_{16}$ = 2.0 |  | SFL6 |
|  | $r_{17}$= -19.953 |  |  |  |
| 52 |  |  | $d_{17}$ = 70.0 | BK7 |
|  | $r_{18}$= 316.23 |  |  |  |
|  |  | $d_{18}$ = 4.0 |  | SSKN8 |
|  | $r_{19}$= -16.079 |  |  |  |
| 50 |  | $d_{19}$ = 2.0 |  | SFL6 |
|  | $r_{20}$= -34.974 |  |  |  |
|  | $r_{21}$= 29.427 |  |  |  |
|  |  | $d_{20}$ = 3.0 |  | SK5 |
|  | $r_{22}$= -199.53 |  |  |  |
| 52 |  |  | $d_{21}$ = 50.0 | BK7 |
|  |  |  | $d_1$ = 2.0 | Luft |
|  | $r_{23}$= -13.725 |  |  |  |
| 51 |  | $d_{22}$ = 2.0 |  | SK11 |
|  | $r_{24}$= 51.955 |  |  |  |
|  |  | $d_{23}$ = 3.0 |  | SF10 |
|  | $r_{25}$= -104.41 |  |  |  |

Table II

| Elément optique | Rayon $r_i$/mm | Epaisseur $d_i$/mm | Distance $d_i$/mm | Type de verre |
|---|---|---|---|---|
| 57.1,57.2 | $r_1$ = 65.879 |  |  |  |
|  |  | $d_1$ = 1.0 |  | SF10 |
|  | $r_2$ = 15.179 | $d_2$ = 1.5 |  | SSK50 |
|  | $r_3$ = -22.876 |  |  |  |
| 56 | $r_4$ = 21.288 | $d_3$ = 3.0 |  | SSKN8 |
|  | $r_5$ = -9.8571 | $d_4$ = 1.0 |  | SF8 |
|  | $r_6$ = -36.256 |  |  |  |
| 52 |  |  | $d_5$ = 37.2 | BK7 |
|  | $r_7$ = 19.248 | $d_6$ = 5. |  | SK11 |
| 47 | $r_8$ = -5.7876 | $d_7$ = 2.0 |  | SFL6 |
|  | $r_9$ = -10.981 |  |  |  |
| 52 |  |  | $d_8$ = 56.83 | SF1 |
|  | $r_{10}$= 77.736 | $d_9$ = 3.0 |  | SK2 |
|  | $r_{11}$= -10.218 |  |  |  |
| 48 | $r_{12}$= -22.227 | $d_{10}$ = 2.0 |  | SF10 |
|  | $r_{13}$= 28.799 | $d_{11}$ = 3.0 |  | SF8 |
|  | $r_{14}$= -285.92 |  |  |  |
| 52 |  |  | $d_{12}$ = 42.99 | SF1 |
|  | $r_{15}$= 330.18 | $d_{13}$ = 4.0 |  | SSK51 |
|  | $r_{16}$= -11.383 | $d_{14}$ = 2.0 |  | SF10 |
|  | $r_{17}$= -258.52 | $d_{15}$ = 4.0 |  | SF1 |
| 49 | $r_{18}$= 196.68 | $d_{16}$ = 4.0 |  | SSKN8 |

Table II   (suite)

| Elément optique | Rayon $r_i$/mm | Epaisseur $d_i$/mm | Distance $d_i$/mm | Type de verre |
|---|---|---|---|---|
| | $r_{19}$= -7.1821 | $d_{17}$ = 2.0 | | SF55 |
| | $r_{20}$= -10.0 | | | |
| 52 | | | $d_{18}$ = 54.16 | SF1 |
| | $r_{21}$= 196.68 | $d_{19}$ = 3.0 | | SK2 |
| | $r_{22}$= -9.7163 | | | |
| 50 | $r_{23}$= -24.76 | $d_{20}$ = 2.0 | | SF1 |
| | $r_{24}$= 20.535 | $d_{21}$ = 3.0 | | F5 |
| | $r_{25}$= -149.62 | | | |
| 52 | | | $d_{22}$ = 38.29 | SF1 |
| | | | $d_1$ = 1.5.. | Luft |
| | $r_{26}$= -18.701 | $d_{23}$ = 1.0 | | SSK51 |
| | $r_{27}$= 4.5973 | $d_{24}$ = 4.0 | | SF1 |
| 51 | $r_{28}$= 38.404 | | | |
| | $r_{29}$= -5.4639 | $d_{25}$ = 2.0 | | Luft |
| | $r_{30}$= -31.623 | $d_{26}$ = 1.0 | | SF10 |

## Table III

| Sous-ensemble N° | N° | Rayon r$_i$/mm | Epaisseur/Distance d$_i$/mm | Type de verre |
|---|---|---|---|---|
| Objectif principal + optique d'injection | | | 2.08 | |
| | 1 | 28.387 | 3.0 | BaF4 |
| | 2 | −14.855 | 1.0 | SF56A |
| | 3 | −35.227 | 0 | |
| | 4 | ∞ | 48.62 | LAKN22 |
| | 5 | ∞ | 0 | |
| | 6 | 20.833 | 4.0 | F5 |
| Optique de transmission | 7 | −7.9433 | 2.0 | SF56A |
| | 8 | −15.510 | 0 | |
| | 9 | ∞ | 53.85 | LAKN22 |
| | 10 | ∞ | 0 | |
| | 11 | 15.51 | 2.0 | SF56A |
| | 12 | 7.9433 | 4.0 | F5 |
| | 13 | −7.9433 | 2.0 | SF56A |
| | 14 | −15.51 | 0 | |
| | 15 | ∞ | 53.85 | LAKN22 |
| | 16 | ∞ | 0 | |
| | 17 | 15.51 | 2.0 | SF56A |
| | 18 | 7.9433 | 4.0 | F5 |
| | 19 | −7.9433 | 2.0 | SF56A |
| | 20 | −15.51 | 0 | |
| | 21 | ∞ | 53.85 | LAKN22 |
| | 22 | ∞ | 0 | |
| | 23 | 21.288 | 2.0 | SSKN8 |
| | 24 | −147.49 | 0 | |
| | 25 | 12.68 | | |

| | | | |
|---|---|---|---|
| 26 | −15.399 | 3.0 | SSKN8 |
| | | 1.0 | SF56A |
| 27 | −258.52 | | |
| 28 | ∞ | 0 | |
| 29 | ∞ | 19.79 | SF10 |
| 30 | −5.233 | 1.4 | |
| 31 | −3.7584 | 2.0 | SF10 |
| 32 | −76.076 | 1.0 | SK11 |
| 33 | −4.5973 | 1.6 | |
| 34 | −13.626 | 1.4 | BK7 |

Objectif d'endoscope ß = 6 angle d'image 45°

Distance de travail d = 45,2mm, grossissement ß = 6x, diamètre de champ visuel 2l = 40mm, angle d'image 45°

34

EP 0 582 148 B1

## Table IV

| Sous-ensemble N° | | Rayon $r_i$/mm | Epaisseur/Distance $d_i$/mm | Type de verre |
|---|---|---|---|---|
| | | | 2.08 | |
| | 1 | 28.387 | 3.0 | BaF4 |
| | 2 | −14.855 | | |
| Objectif | | | 1.0 | SF56A |
| principal | 3 | −35.227 | | |
| + optique | | | 0 | |
| d´injection | 4 | ∞ | | |
| | | | 48.62 | LAKN22 |
| | 5 | ∞ | | |
| | | | 0 | |
| | 6 | 20.833 | | |
| | | | 4.0 | F5 |
| | 7 | −7.9433 | | |
| | | | 2.0 | SF56A |
| | 8 | −15.510 | | |
| | | | 0 | |
| | 9 | ∞ | | |
| | | | 53.85 | LAKN22 |
| | 10 | ∞ | | |
| | | | 0 | |
| | 11 | 15.51 | | |
| Optique de | | | 2.0 | SF56A |
| transmission | 12 | 7.9433 | | |
| | | | 4.0 | F5 |
| | 13 | −7.9433 | | |
| | | | 2.0 | SF56A |
| | 14 | −15.51 | | |
| | | | 0 | |
| | 15 | ∞ | | |
| | | | 53.85 | LAKN22 |
| | 16 | ∞ | | |
| | | | 0 | |
| | 17 | 15.51 | | |
| | | | 2.0 | SF56A |
| | 18 | 7.9433 | | |
| | | | 4.0 | F5 |
| | 19 | −7.9433 | | |
| | | | 2.0 | SF56A |
| | 20 | −15.51 | | |
| | | | 0 | |
| | 21 | ∞ | | |
| | | | 53.85 | LAKN22 |
| | 22 | ∞ | | |
| | | | 0 | |
| | 23 | 19.5208 | | |
| | | | 3.0 | SSKN8 |
| | 24 | − 9.60378 | | |

| | | 1.0 | SF56A |
| 25 | −40.4719 | | |
| 26 | 11.1552 | 0 | |
| | | 2.0 | F5 |
| 27 | −250.0 | 0 | |
| 28 | $\infty$ | | |
| | | 17.11 | SF10 |
| 29 | $\infty$ | 0.5 | |
| 30 | −6.1514 | | |
| | | 2.0 | SF10 |
| 31 | −3.25855 | 1.0 | SK11 |
| 32 | 69.5948 | | |
| | | 1.0 | |
| 33 | −3.37032 | 1.0 | BaF4 |
| 34 | −11.0651 | | |
| | | 1.0 | |
| 35 | −3.79897 | 1.5 | BK7 |
| 36 | −8.59761 | | |

Distance de travail d = 46,0mm, grossissement ß = 8x, diamètre de champ visuel 2l = 52mm, angle d´image 60°

FIG. 1a

FIG. 2a

# FIG.1b

EP 0 582 148 B1

# FIG.2b

EP 0 582 148 B1

## FIG. 3a

## FIG. 3b

## FIG. 3c

FIG.4

EP 0 582 148 B1

## FIG.5

## FIG.6

FIG. 7

# FIG. 8

EP 0 582 148 B1